(19) Europäisches Patentamt
European Patent Office
Office européen des brevets

(11) **EP 4 703 021 A1**

(12) **EUROPEAN PATENT APPLICATION**

(43) Date of publication:
**04.03.2026  Bulletin 2026/10**

(21) Application number: **24197117.5**

(22) Date of filing: **28.08.2024**

(51) International Patent Classification (IPC):
***B01D 19/00*** (2006.01)     ***B01D 21/30*** (2006.01)
***B01D 21/34*** (2006.01)

(52) Cooperative Patent Classification (CPC):
**B01D 19/0063; B01D 19/0068; B01D 21/30**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Envetec Sustainable Technologies
Limited
Birdhill, Co. Tipperary V945K02 (IE)**

(72) Inventors:
• **MCCRAITH, Alan
Birdhill, Co. Tipperary V945K02 (IE)**
• **PURCELL, Patrick
Islip Terrace, New York 11752 (US)**

(74) Representative: **Edson, Russell Gregory
Withers & Rogers LLP
2 London Bridge
London SE1 9RA (GB)**

(54)   **WASTE PROCESSING APPARATUS AND METHOD**

(57)     A waste processing apparatus is disclosed which includes a waste processing vessel for holding waste and treatment fluid during processing, and a fluid delivery system for delivering a treatment fluid to the waste processing vessel. The fluid delivery system comprises a settling vessel located upstream of the waste processing vessel, the settling vessel defining an internal volume configured to retain a treatment fluid, a first fluid transfer means arranged and configured to transfer the treatment fluid from a fluid reservoir to the settling vessel; and a second fluid transfer means arranged and configured to deliver treatment fluid from the settling vessel to the waste processing vessel. The fluid delivery system is configured such that treatment fluid is retained in the settling vessel for a period of time prior to delivery to the waste processing vessel.

FIG. 6A

EP 4 703 021 A1

**Description**

Technical Field

The present disclosure relates to the processing of biohazardous waste. In particular, the disclosure relates to the treatment of waste to prepare it to enter a recycling process. More particularly, the disclosure relates to the disinfection and shredding of biohazardous waste prior to recycling.

Background to the Invention

Currently, biohazardous waste generated from various different sources is typically autoclaved, incinerated and ultimately sent to landfill. As a result, there is currently little to no known recycling of biohazardous waste globally, due to a lack of availability of suitable treatment processes.

More generally, treatment of waste from various sources requires different levels of treatment in order for it to be further processed in any one of various known recycling processes.

Generally, recycling processes are sensitive to contaminants and some degree of cleaning of waste materials may be necessary prior to entering a recycling process, for example by using some form of treatment fluid.

Delivering treatment fluids to waste treatment systems such as recycling processes can be difficult to control and cumbersome for operators.

There exists a need for improved processes and apparatus for delivering treatment fluids to waste treatment systems in which waste materials can be treated more effectively and efficiently in preparation for recycling.

Summary of the Invention

The invention seeks to address, among others, the issue of gas bubbles introduced into, or induced in, a liquid during delivery of the liquid into a waste processing apparatus. Such gas bubbles can render the task of accurately measuring an amount of liquid being delivered into the apparatus more difficult. The invention presents means and methods for settling gas bubbles out of the liquid. This enables an accurate measurement of the volume of liquid to be made prior to delivery of liquid into working or processing parts or zones of the waste processing apparatus. The invention provides means and methods of retaining a liquid in a settling chamber to allow gas bubbles to settle out of the liquid for a period of time. The period of time may be predetermined or may be determined based upon outputs of one or more sensors in the system. The sensors may indicate when a sufficient or desired amount of bubbles have settled out of the liquid. The decision to deliver the liquid into the waste processing zone or area of the waste processing apparatus may be made based upon the outputs of such sensors, or based upon an amount of time having elapsed.

In addressing these and other matters relating to waste processing apparatus, the following aspects are provided.

According to an aspect of the invention, there is provided a waste processing apparatus, comprising any or all of the following features: a waste processing vessel for holding waste and treatment fluid during processing; and a fluid delivery system for delivering a treatment fluid to the waste processing vessel, the fluid delivery system comprising any or all of the following features: a settling vessel located upstream of the waste processing vessel, the settling vessel defining an internal volume configured to retain a treatment fluid; a first fluid transfer means arranged and configured to transfer the treatment fluid from a fluid reservoir to the settling vessel; and a second fluid transfer means arranged and configured to deliver treatment fluid from the settling vessel to the waste processing vessel; wherein the fluid delivery system is configured such that treatment fluid is retained in the settling vessel for a period of time prior to delivery to the waste processing vessel.

The waste processing apparatus may comprise a blade arrangement provided in the waste processing vessel, for shredding waste.

The waste processing vessel may comprise a waste processing zone, in which treatment fluid can be retained, such that waste provided to the waste processing vessel can be shredded by the blade arrangement while immersed in treatment fluid in the waste processing zone.

The fluid delivery system further may comprise a sensor arrangement configured to measure a parameter associated with the fluid in the settling vessel and to generate a signal based on the parameter.

The sensor arrangement may comprise one or more level sensors.

The sensor arrangement may comprise a plurality of level sensors.

The plurality of level sensors may be vertically aligned relative to each other with respect to a base region of the settling vessel.

The one or more level sensors may be capacitive sensors.

The settling vessel may comprise one or more slots in a wall thereof, each of the one or more slots configured to receive a respective level sensor of the one or more level sensors.

The settling vessel may comprise at least two slots in a side wall thereof, each slot may be configured to receive a respective level sensor.

The at least two slots may be arranged such that the respective level sensors are vertically aligned relative to each other with respect to the base region of the settling vessel.

The at least two slots may be arranged such that the respective level sensors are vertically aligned relative to each other with respect to a base region of the settling vessel.

The first fluid transfer means and/or the second fluid transfer means may comprise a valve moveable to control flow of treatment fluid into and/or out of the settling vessel.

The fluid delivery system may further comprise a controller configured to control flow of treatment fluid into and out of the settling vessel based on the signal from the sensor arrangement.

The controller may be configured to control one or more valves of the first and/or second fluid transfer means.

The controller may be configured to determine whether a predetermined condition associated with the fluid in the settling vessel has been met based on the signal from the sensor arrangement and to generate a first output to instruct the second fluid transfer means to transfer the fluid from the settling vessel to the waste processing vessel based at least upon a determination that the predetermined condition has been met

The predetermined condition may comprise a minimum settling time over which the treatment fluid has been present in the settling vessel.

The waste processing vessel may be configured to perform a waste processing cycle for a cycle time, and the minimum settling time may be greater than or equal to the cycle time.

The waste processing vessel may be configured to perform one or more waste processing cycles. The controller may be configured to generate the first output in response to a determination that a fluid dispensing step of a waste processing cycle has been reached.

The controller may be configured to identify the presence of a treatment fluid in the settling vessel based on the signal from the sensor arrangement. The controller may be configured to determine whether an elapsed time from identifying the treatment fluid in the settling vessel is greater than or equal to the minimum settling time.

The controller may be configured to generate the first output based upon a further determination that a predetermined volume of fluid is present in the settling vessel.

The predetermined condition may comprise a predetermined volume of treatment fluid present in the settling vessel.

The controller may be configured to determine whether a volume of treatment fluid in the settling vessel is below the predetermined volume based on the signal from the sensor arrangement and in response thereto generate a second output to instruct the first fluid transfer means to deliver further treatment fluid into the settling vessel from the fluid reservoir.

The controller may be configured to generate the second output prior to the waste processing vessel performing a waste processing cycle.

The controller may be configured to determine whether the volume of the treatment fluid in the settling vessel is less than the predetermined volume based on the signal from the sensor after generating the second output, and in response thereto generate a fourth output to indicate that the fluid reservoir is empty.

The controller may be configured to determine whether the volume of the treatment fluid in the settling vessel is less than the predetermined volume based on the signal from the sensor after generating the second output, and in response thereto generate a fourth output to indicate that the fluid reservoir is empty.

The waste processing vessel may be configured to perform one or more waste processing cycles. The controller may be configured to generate the first output in response to a determination that a fluid dispensing step of a waste processing cycle has been reached.

The predetermined condition may comprise a maximum rate of change of volume of treatment fluid in the settling vessel with respect to time.

The controller may be configured to determine whether a rate of change of volume of the treatment fluid in the settling vessel is at or below the maximum rate of change based on the signal from the sensor arrangement and generate the first output in response thereto.

The controller may be configured to generate a third output to instruct the first fluid transfer means to draw a predetermined volume of treatment fluid from the fluid reservoir into the settling vessel.

The controller may be configured to generate the third output after generating the first output.

The controller may be configured to detect that the treatment fluid has been transferred from the settling vessel to the waste processing vessel and in response thereto generate the third output.

The waste processing vessel may be configured to perform a waste processing cycle. The controller may be configured to generate the third output prior to the waste processing vessel performing a waste processing cycle.

The controller may be configured to determine whether the fluid reservoir is empty and in response thereto generate a fourth output.

The controller may be configured to determine whether the volume of the treatment fluid in the settling vessel is less than the predetermined volume based on the signal from the sensor arrangement after generating the third output, and in response thereto generate a fourth output to indicate that the fluid reservoir is empty.

The first fluid transfer means may comprise a pump operable to draw fluid from the reservoir to the settling vessel. The pump may be operable in response to an output (e.g. the third output and/or the second output) from the controller.

The second fluid transfer means may comprise a pump operable to draw fluid from the settling vessel to the waste processing vessel. The pump may be operable in response to an output (e.g. the first output) from the controller.

The first fluid transfer means may comprise a valve moveable between an open position and a closed position. The controller may be configured to generate an output (e.g. the third output and/or the second output) to move the valve to the open position so as to deliver treatment fluid from the fluid reservoir to the settling vessel. The valve may be a solenoid valve.

The first fluid transfer means may comprise a fluid conduit arranged to provide fluid communication between the fluid reservoir and the settling vessel. One or more valves and/or a pump may be provided along the fluid conduit of the first fluid transfer means.

The second fluid transfer means may comprise a valve moveable between an open position and a closed position. The controller may be configured to generate the first output to move the valve to the open position so as to deliver treatment fluid from the settling vessel to the waste processing vessel. The valve may be a solenoid valve.

The waste processing vessel may be arranged relative to the settling vessel so that fluid can fall under gravity from the settling vessel to the waste processing vessel when the controller generates the first output.

The second fluid transfer means may comprise a fluid conduit arranged to provide fluid communication between the settling vessel and the waste processing vessel. One or more valves may be provided along the fluid conduit of the second fluid transfer means.

The treatment fluid may comprise a gas-liquid mixture which can release bubbles in the settling vessel.

According to an aspect of the invention, there is provided method of delivering a treatment fluid to a waste treatment system, the method comprising any or all of the following steps: transferring a treatment fluid from a fluid reservoir to a settling vessel configured to retain the treatment fluid; retaining the treatment fluid in the settling vessel for a period of time; transferring the treatment fluid from the settling vessel to a waste treatment system after the period of time has elapsed.

The method may include measuring a parameter associated with the fluid in the settling vessel.

The method may include using a sensor arrangement to measure a parameter associated with the fluid in the settling vessel.

The step of allowing the treatment fluid to settle may include determining whether a predetermined condition associated with the fluid in the settling vessel has been met based on the measured parameter.

The method may include transferring the treatment fluid from the settling vessel to a waste treatment system when it is determined that the predetermined condition has been met.

The method may be performed by a controller.

Any or all of the features of the previous aspect may be included in the method.

According to an aspect of the invention, a computer-readable medium is provided, the computer-readable medium comprising instructions which, when executed by a processor, cause the processor to perform any or all of the following: control a first fluid transfer means to transfer a treatment fluid from a fluid reservoir to a settling vessel configured to retain the fluid; retain the treatment fluid in the settling vessel for a period of time; and control a second fluid transfer means to transfer the treatment fluid from the settling vessel to a waste processing vessel after the period of time has elapsed.

The computer-readable medium may comprise instructions which, when executed by a processor, cause the processor to measure a parameter associated with the fluid in the settling vessel.

The computer-readable medium may comprise instructions which, when executed by a processor, cause the processor to use a sensor arrangement to measure a parameter associated with the fluid in the settling vessel.

The computer-readable medium may comprise instructions which, when executed by a processor, cause the processor to determine whether a predetermined condition associated with the fluid in the settling vessel has been met based on a measured parameter associated with the fluid in the settling vessel.

The computer-readable medium may comprise instructions which, when executed by a processor, cause the processor to control a second fluid transfer means to transfer the treatment fluid from the settling vessel to a waste processing vessel when the predetermined condition has been met.

Any or all of the features of the previous aspects may be applied to the computer-readable medium.

According to a further aspect of the invention, there is provided a fluid delivery system for delivering a treatment fluid to a waste treatment system, the fluid delivery system comprising any or all of the following features: a settling vessel located upstream of the waste processing vessel, the settling vessel defining an internal volume configured to retain a treatment fluid; a first fluid transfer means arranged and configured to transfer the treatment fluid from a fluid reservoir to the settling vessel; a second fluid transfer means arranged and configured to deliver treatment fluid from the settling vessel to a waste treatment system; wherein the fluid delivery system is configured to retain treatment fluid in the settling vessel for a period of time prior to delivering the treatment fluid to the waste processing vessel.

Any or all of the features of the previous aspects may be applied fluid delivery system.

Brief Description of the Drawings

Further features and advantages of the present disclosure will become apparent from the following description of embodiments thereof, presented by way of example only, and by reference to the drawings, in which:

Figure 1 shows a waste processing apparatus according to one example;
Figure 2 shows the waste processing apparatus from an opposite angle;
Figure 3 shows a waste processing vessel of the disclosure;
Figures 4A and 4B show a waste processing vessel of the disclosure;
Figures 5A and 5B show a fluid delivery means for the apparatus of Figures 1 and 2;
Figure 6A shows a schematic of a fluid delivery system of the disclosure;
Figure 6B shows a settling vessel of the disclosure;
Figure 6C shows a method of delivering a treatment fluid to a waste treatment system;
Figures 7A to 7C show the waste processing apparatus in different operating configurations;
Figure 8 shows a schematic diagram of a waste processing apparatus;
Figure 9 shows an example of a method of controlling a waste processing apparatus;
Figure 10 shows an example of a method of controlling the fluid delivery system;
Figures 11A and 11B show an example of a waste cycle process;
Figures 12A and 12B show an example of a validation cycle process; and
Figure 13 shows an example of a reject cycle process.

Detailed Description

A large amount of biologically hazardous waste is generated from different sources, including but not limited to clinical facilities, diagnostic laboratories, and research institutes. This waste requires specialised treatment. Such treatment typically occurs off-site and therefore also requires specialised transportation and handling ahead of treatment, which can increase costs, poses a potential contamination or public health risk and increases the carbon footprint. Treatment of hazardous waste, biohazardous medical, or other regulated waste, in particular, often requires energy intensive processes, such as transport for off-site processing, along with autoclaving and/or incineration, whose emissions, water and energy usage, and environmental impact can be improved upon.

A particular aspect of biologically hazardous (biohazardous) waste is that the risks relating to contaminants present in or on the waste need to be handled throughout its processing, until such time as it is considered safe for contact with humans or the environment.

The inventors have identified particular areas for improvement in the processing of contaminated, hazardous or biohazardous waste. More particularly, the inventors have developed a technology that shreds and disinfects biohazardous waste at source and hence facilitates the recycling of these waste streams. In doing so, the disclosure also combines several of the processes that take place separately in a recycling work-flow, hence increasing the downstream usability of the treated end product produced by the disclosure.

The disclosed systems and its various aspects are particularly suited to use with the vast array of different biohazardous waste products that are produced in laboratories. Such waste streams may contain glass, paper, polymers, blood, urine, sharps, PPE, etc. and hence the blade arrangement needs to cope with a range of hard and soft materials, some being pliable and malleable, others being hard and brittle. None of these materials can be recycled while they are considered to be of a biohazardous nature. To reliably disinfect biohazardous waste, the waste needs to be shredded sufficiently to ensure that a treatment chemical comes into contact with the biohazardous materials. Once the initially biohazardous waste is sufficiently treated to be considered non-hazardous and safe for handling and transportation, it can be sent to recycling facilities for separation and pelletisation. The disclosed system facilitates recycling, as the waste processed in the system can be made safe. Combined disinfection or decontamination and shredding is an advantage to downstream recyclers as they can skip several steps in the usual known processes at recycling plants, with the output from blade arrangements and systems as disclosed herein.

As used herein, the term "biohazardous waste" (also commonly referred to as "infectious waste") takes its usual meaning in the art and relates to waste that has been contaminated with potentially infectious agents, or other materials that are deemed to be a threat to either public health or to the environment. For example, biohazardous waste includes medical waste generated in laboratory or clinical settings (such as hospitals), which has been contaminated with blood, body fluids, and/or cell lines from humans or animals. Such waste can also sometimes fall within the terms medical waste or regulated waste, depending on the different national regulatory definitions.

**Waste Processing Apparatus**

The waste processing apparatus described herein provides a number of advantageous features and aspects. One advantageous feature is the system being able to perform shredding of waste while the waste is submersed or immersed in a reservoir of the treatment fluid, which provides simultaneous treatment, such as sterilisation or disinfection, simultaneously with the shredding of the waste. This can reduce overall processing times and improve the reliability of the shredding and disinfection or sterilisation, or other cleansing process. The treatment fluid may comprise liquid, such as water, and may comprise a treatment agent. The treatment fluid may be in the form of a homogenous liquid mixture or a solution. The treatment agent may be provided in solid, liquid or gel form and may be combined with a diluent, which may be a liquid diluent such as water. As described herein, the term "diluent" takes its usual meaning in the art and relates to a substance that is added to dilute a solution or a mixture. The treatment fluid may comprise a gaseous component in some arrangements. The treatment fluid may comprise liquid and gaseous components, e.g. the treatment fluid may include liquid particles suspended in a gas (e.g. an aerosol) or gas particles suspended in a liquid, or a mixture of liquid and gas components. In one arrangement, the treatment fluid may include gaseous chlorine dioxide. The treatment fluid may include aqueous chlorine dioxide. The treatment fluid may include chlorine dioxide gas dissolved in a liquid, e.g. water.

As used herein, the term "solution" relates to liquid mixture in which the minor component(s) (i.e., the treatment agent) is uniformly distributed throughout the major component (i.e., the diluent). The treatment agent in the form of a solution may also be referred to as the treatment solution. The diluent may comprise, consist essentially of or preferably consist of, water. The treatment solution may be acidic. The pH of the treatment solution may be in the range of from about 2 to about 6.

Advantageously, the process may use peracetic acid ($CH_3CO_3H$, also commonly referred to as peroxyacetic acid) and hydrogen peroxide in the waste shredding and disinfecting process. This and other such fluids as described herein can improve the efficiency and efficacy of the treatment of the waste simultaneously with the shredding process. The design of the waste processing apparatus is particularly suited to the treatment of hazardous waste, such as biohazardous waste or other waste containing harmful or toxic substances or infectious agents, from which a user must be protected before during and after processing of the waste. Advantageous aspects of the design of the blade arrangement used in the process for improved shredding efficiency and flexibility are also described. Advantageous control schemes and methods of operation of the process are also described, which improve the efficacy, user experience and user safety and overall process efficiency of the device.

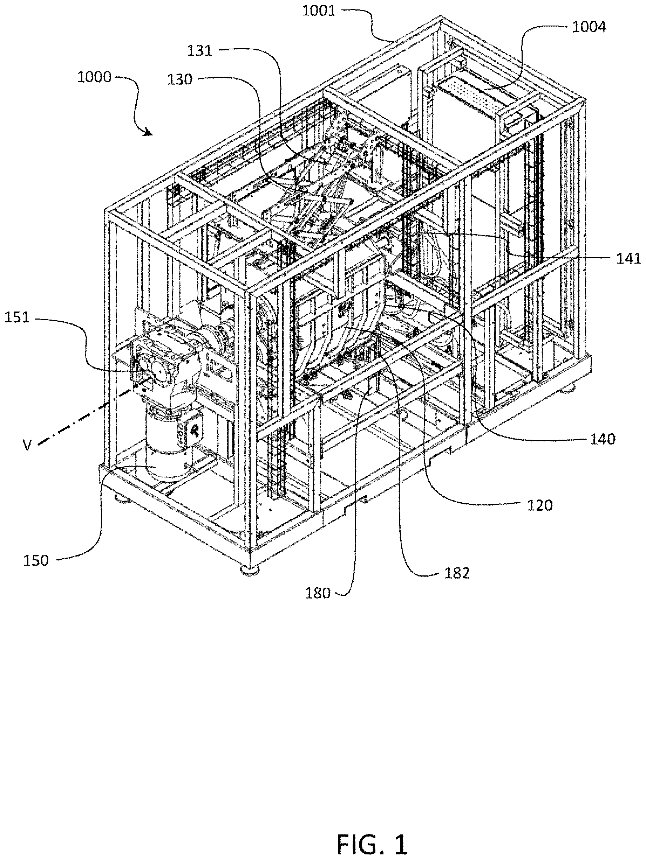

With reference to Figure 1, there is shown a waste processing apparatus 1000 according to the disclosure. The apparatus comprises a chassis 1001, comprising a series of struts and beams arranged to mechanically support and surround the working components of the waste processing apparatus. A plurality of wall portions, such as panels, doors or plates may be arranged on or around the chassis. In this manner an enclosure for the working components of the apparatus can be provided. A waste processing vessel 120 may be provided within an enclosure, such as that provided by the chassis 1000. There may further be provided a control system enclosure 1004, which may include power electronics, signal and power distribution modules and computing and/or processing devices for the control of the apparatus. A closure mechanism 130 may be provided for moving the closure arrangement, such as a lid, which may be termed a press-plate, for closing a waste receiving opening provided in the waste processing vessel 120. The closure mechanism may comprise a closure mechanism actuator 131, which may be a linear or rotational actuator. It may further comprise a closure mechanism sensor for sensing a position of the closure mechanism. This can enable the detection of a position of the lid or press plate 170 to verify whether it is in an open or a closed position. The waste processing vessel 120 may be rotatable about a vessel rotation axis V. Vessel displacement means may be provided to displace vessel 120 between different operational positions. Vessel rotation drive means 140 may be provided to drive the vessel 120 in rotation about an axis V. The vessel 120 may be moved between different configurations to enable different operations, such as loading of the vessel, closure of the vessel for processing, and emptying of the vessel of processed waste and treatment fluid (e.g. a fluid dispensing step). This movement between different configurations may be provided by rotation about the axis V. However, other displacement means and types of displacement of the vessel between the different positions may be envisaged, such as linear, translational, a combination of rotation and translation among others within the knowledge of the skilled reader. The vessel drive means 140, which may be rotational drive means, may be mounted to a part of the chassis 1000 and may drive the vessel via a gearbox 141, which may also be mounted to a part of the chassis 1000. When rotatable, the axis of rotation of the vessel may also be the same axis about which a drive input for a blade arrangement provided in the vessel is provided, as will be described in relation to later figures. Blade rotation drive means 150 may be provided in the form of a motor. The motor 150 may drive the blades in rotation via a gearbox 151. One or more of motors 140 and 151 may be mounted with its axis of rotation perpendicular to the axis of rotation of the waste processing vessel 120 and/or the axis of rotation of the blade arrangement. This can enable a more compact overall arrangement of the waste processing apparatus within its enclosure.

Whether rotationally mounted and rotationally displaceable or not, it can be advantageous to lock the waste processing vessel 120 in position during the waste processing cycle. This inhibits vibration of the waste processing vessel 120 which may be caused by rotation of the blades. To this end, positional locking means may be provided to the vessel to retain the vessel in place within the chassis 1001 and prevented generally from rotational and/or translational movement during the processing cycle. Examples of positional locking means may include friction brakes, bolts clamps or other means for retaining the vessel in place.

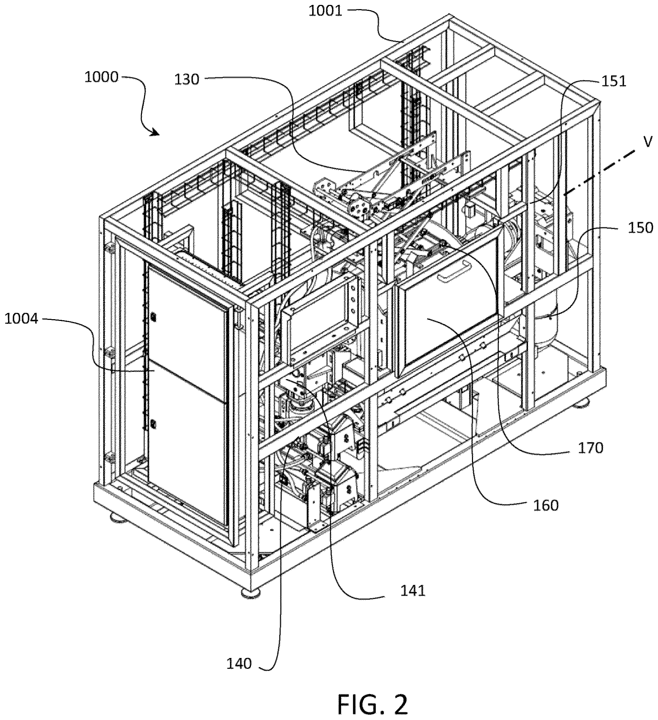

Turning to Figure 2, a waste loading hatch 160 may be provided which can be opened for loading of the waste processing vessel 120 with waste and which may be closed in order to enclose the waste processing vessel during a waste processing cycle of the apparatus. This can help to protect users from leakage or spillage of contaminants or treatment fluids during the processing cycle. In the present example, a handle 161 is provided on the waste loading hatch 160 to assist a user with manually opening the hatch 160. In other examples, the opening of the hatch may be automated e.g. to allow a robot or other automated systems or handlers to load the waste processing vessel 120.

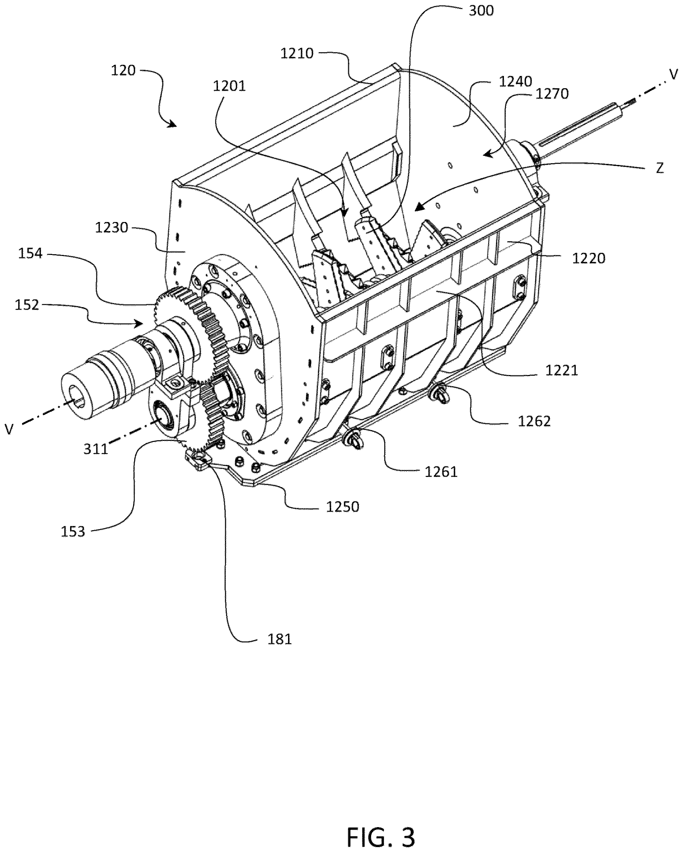

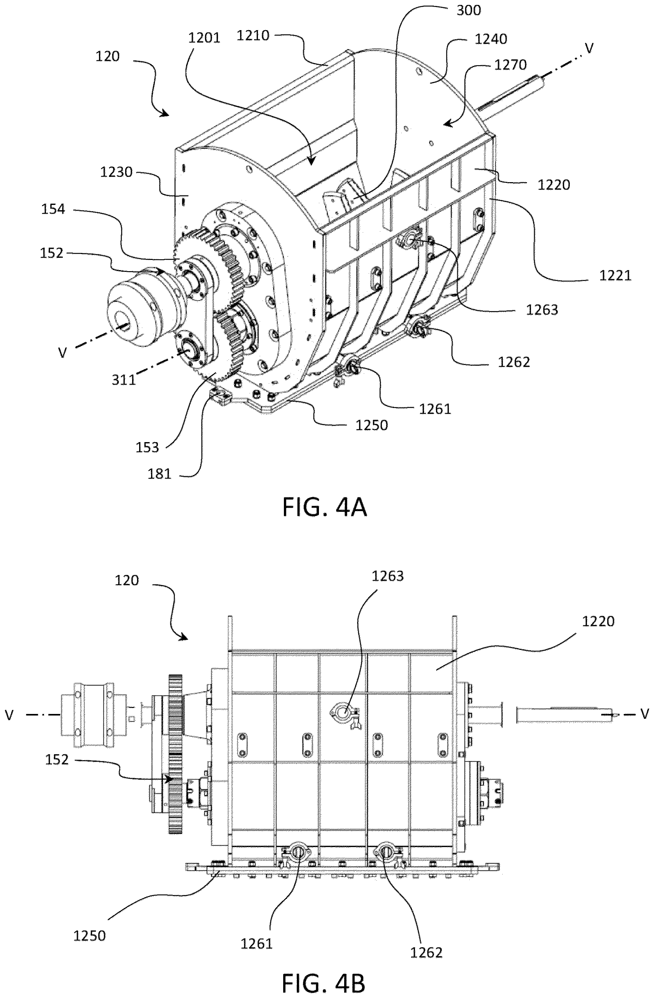

Figures 3, 4A and 4B illustrates a waste processing vessel 120 outside of the enclosure illustrated in Figures 1 and 2. The waste processing vessel may comprise at least one wall 1210, 1220, arranged to form a waste processing chamber 1201 into which waste can be placed for fluid treatment by the apparatus, as well as shredding in some arrangements. The at least one wall may comprise a first side wall 1210, a second side wall 1220, a first end wall 1230 and a second end wall 1240. One or more of the at least one walls may comprise one or more strengthening ribs 1221 to provide strength to the wall to resist shredding and compression forces which may be applied to the wall during rotation of a blade arrangement 300 located in the waste processing vessel 120. A bottom wall 1250 may be provided. The waste processing chamber 1201 may be provided in the form of a closed-bottomed container, in which an amount of fluid may be retained such that waste residing in a bottom of the fluid can be maintained in an immersed state under an amount of fluid provided to the waste processing vessel 120.

The waste processing vessel 120 may be provided with one or more ports 1261, 1262, 1263 for receiving process validation samples. The one or more ports 1261, 1262 may be arranged to be externally accessible with respect to the waste processing vessel, so that the user can access the ports to install and/or remove process validation samples from the waste processing vessel. It can be advantageous to have the one or more ports 1261, 1262, 1263 located in a waste processing zone of the waste processing vessel 120. The one or more validation ports 1261, 1262, 1263 may be arranged to provide an opening through one or more walls of the waste processing vessel, such as wall 1220. The one or more validation ports 1261, 1262, 1263 provide exposure of process validation samples to the interior of the vessel 120. The process validation samples located in the ports 1261, 1262, 1263 may be exposed to the treatment fluid in the vessel 120 so as to provide an indicator as to the efficacy of the treatment fluid. The samples can provide information regarding the efficacy of the treatment in various regions of the vessel 120. The samples may include a biological indicator. The term "biological indicator" relates to microorganisms which are suitable for determining the efficacy of the waste shredding and liquid treatment process and includes spores produced by such microorganisms.

In the figures, a plurality of validation ports 1261, 1262, 1263 are provided. The plurality of validation ports 1261, 1262, 1263 are spaced apart from one another so as to provide an opening into different locations of the vessel 120 interior, and to different regions of the waste processing zone Z. As can be seen in Figures 4A and 4B, some of the ports 1261, 1262, 1263 may be spaced apart from one another in a direction of the waste receiving opening 1270, so as to provide one or more openings into different height-wise positions within the vessel 120 interior when the vessel is oriented in the shredding position, i.e. with the waste receiving opening 1270 oriented upwards.

The one or more ports may include one or more lower ports 1261, 1262. The one or more lower ports 1261, 1262 may be provided in a lower region of such wall so as to be located in a region of the waste processing vessel in the waste processing zone, which may be a lower region of the waste processing vessel 120 when it is oriented in its waste processing position. As shown in Figures 4A and 4B, the one or more ports may include one or more upper ports 1263. The one or more upper ports 1263 may be provided in an upper region of a wall of the vessel 120. The one or more upper ports 1263 are positioned so as to be above the lower ports 1261, 1262 when the waste processing vessel 120 is oriented in its waste processing position. In Figure 3, only two lower ports 1261, 1262 are provided. In Figures 4A and 4B, two lower ports 1261, 1262 are provided and one upper port 1263 is provided. It will be appreciated that any number or arrangement of ports may be implemented that provides exposure of one or more process validation samples to various locations within the vessel 120. In some arrangements, only one of the upper and lower ports may be included. The arrangement of the validation ports shown in the figures, such as being vertically spaced apart, provides a means for testing the efficacy of the fluid treatment process of the waste, whilst also providing a means for testing the quality of the atmosphere including any infectious aerosol contaminants, such as aerosolised particles from the waste material, in the vessel 120 prior to opening the vessel 120 and releasing any gases or airborne material therein to the atmosphere.

As noted above, the waste processing apparatus 1000 may include a blade arrangement 300 in some arrangements. The blade arrangement 300 may be driven in rotation about a blade rotation axis 311, optionally via a gear arrangement 152. Gear arrangement 152 may comprise first 153 and second 154 gears arranged to transfer a drive from the drive source such as the drive source 152 the blade arrangement 300. In one arrangement as shown, the vessel rotation axis V may be substantially parallel to the blade rotation axis 311. The gear arrangement 154 may be arranged to transfer a drive between the vessel rotation axis V and the blade rotation axis B. As can be seen, drive for both the blade arrangement 300 and rotation of the waste processing vessel 120 may be provided on the same axis, such that the waste processing vessel is arranged to rotate around a same axis V along which the drive provided for driving the blade arrangement 300.

The waste processing vessel 120 comprises a waste processing zone Z. The waste processing zone Z is an area in the waste processing vessel 120 in which waste is shredded and exposed to a treatment fluid and/or to the treatment process. In the figures, the waste processing zone Z is defined by the interior of the waste processing vessel 120. The waste processing zone Z is configured to retain the treatment fluid. It will be appreciated that in alternative arrangements, the waste processing vessel 120 may include separate chambers. In this case, the waste processing zone Z is defined as the chamber in which the waste is shredded and exposed to the treatment fluid.

When the vessel is in its operating state, at least a portion of the blade arrangement 300, when included, is submerged in the treatment fluid in the waste processing zone Z. The vessel 120 may, for example, be filled or partially filled with treatment liquid to a level. As will become apparent, therefore, waste can be shredded in the zone Z whilst immersed in treatment fluid.

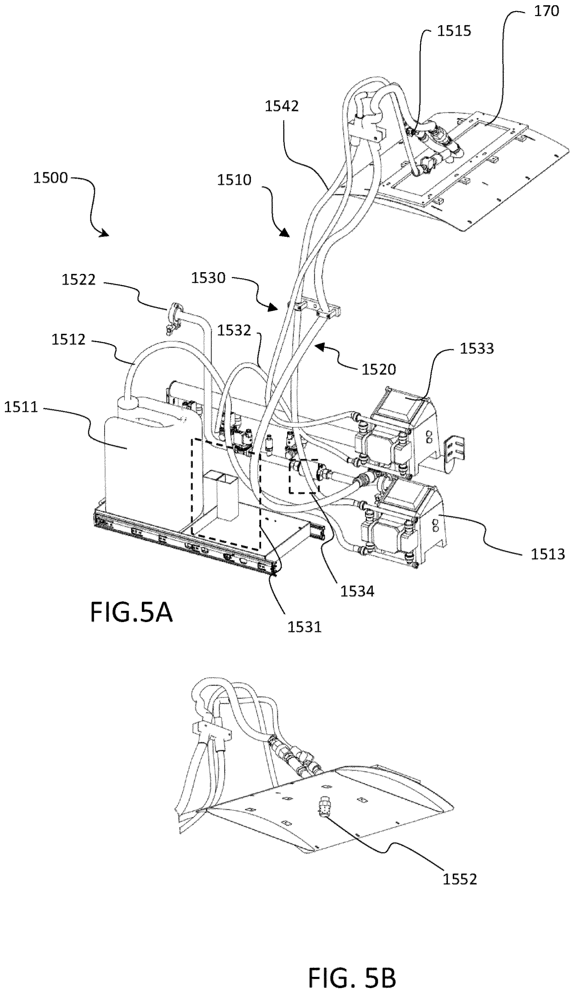

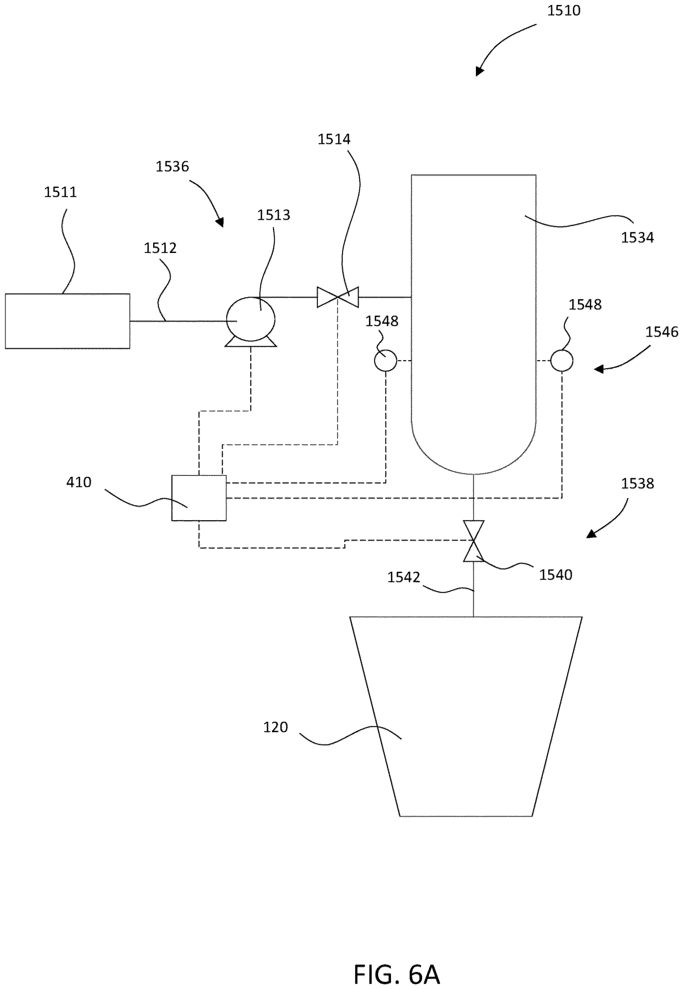

Figure 5A illustrates a fluid delivery means 1500, which may be integrated into the waste processing apparatus 1000. The delivery means 1500 may include a fluid delivery system 1510. The fluid delivery system 1510 may be for delivering a treatment fluid to a waste treatment system. The waste treatment system may be any system configured to treat waste with a treatment fluid. In the illustrated arrangement, the waste treatment system includes the waste processing apparatus 1000 as set forth above. The fluid delivery system 1510 may be configured to deliver treatment fluid to the waste processing vessel 120. The treatment fluid may be as discussed herein, for example, chemical cleaning fluid, disinfectant, sterilisation fluid, a detergent or any other such treatment fluid. An example arrangement of a fluid delivery system 1510 is shown in more detail in Figure 6A.

The fluid delivery system 1510 may include a settling vessel 1534. The settling vessel 1534 is represented schematically by dashed lines in Figure 5A to enable features behind it in that view to be seen. It will be appreciated that the settling vessel 1534 may be located in any suitable position of the fluid delivery system 1510. The settling vessel 1534 defines an internal volume configured to retain the treatment fluid. The settling vessel 1534 may include a series of walls that define an enclosure having a chamber therein for retaining the treatment fluid. In some arrangements, the settling vessel 1534 may have an open end such that the treatment fluid is exposed to atmosphere when located therein. In alternative arrangements, the walls of the settling vessel 1534 may be arranged to completely surround the internal volume. In such an arrangement, the settling vessel 1534 may include a vent system (not shown) for removing any gas generated by or released from the treatment fluid.

The fluid delivery system 1510 may include a first fluid transfer means 1536 arranged and configured to transfer the treatment fluid from a treatment fluid reservoir or container 1511 to the settling vessel 1534. In some arrangements, the fluid reservoir 1511 forms part of the fluid delivery system 1510. The first fluid transfer means 1536 may include a pumping means 1513 (e.g. a pump) operable to draw fluid from the reservoir 1511 to the settling vessel 1534. Treatment fluid pumping means 1513 may be any suitable form of pump, but is advantageously a diaphragm pump. The first fluid transfer means 1536 may include a fluid control means 1514, such as a valve moveable between an open position and a closed position. The valve 1514 is not shown in Figure 5A, but may be located downstream of (or upstream of) the pumping means 1513, as shown in Figure 6A. In the open position, the fluid reservoir 1511 is in fluid communication with the settling vessel 1534 via the first fluid transfer means 1536. In the closed position, the fluid reservoir 1511 is not in fluid communication with the settling vessel 1534. The valve 1514 may be a solenoid valve or may be controlled by a solenoid. The pump 1513 may be upstream or downstream of the valve 1514. The first fluid transfer means 1536 may include a fluid conduit 1512 arranged to provide fluid communication between the fluid reservoir 1511 and the settling vessel 1534 so as to transfer treatment fluid therebetween. One or more valves and/or pumps may be provided along the fluid conduit 1512. The fluid conduit 1512 may be a flexible or solid pipe or tube, for example.

The fluid delivery system 1510 may include a second fluid transfer means 1538 arranged and configured to deliver the treatment fluid from the settling vessel 1534 to a waste treatment system, e.g. the waste processing vessel 120 of the waste processing apparatus 1000. The second fluid transfer means 1538 may include any or all of the features of the first fluid transfer means 1536. The second fluid transfer means 1538 may include a fluid control means, such as a valve 1540 moveable between an open position and a closed position. The valve 1540 is not shown in Figure 5A, but may be located upstream of the waste treatment system as illustrated schematically in Figure 6A. The valve 1540 is used in the arrangement of Figure 5B to control flow through a spray nozzle 1552. In the open position, the settling vessel 1534 is in fluid communication with the waste treatment system via the second fluid transfer means 1538. In the closed position, the settling vessel 1534 is not in fluid communication with the waste treatment system. The second fluid transfer means 1538 may include a fluid conduit 1542 arranged to provide fluid communication between the settling vessel 1534 and the waste treatment system. In some arrangements, the waste processing vessel 1534 may be arranged relative to the waste treatment system (e.g. the waste treatment vessel 120) such that fluid can fall under gravity from the settling vessel 1534 to the waste treatment system. In one arrangement, no fluid conduit 1542 may be provided, and rather a base region 1548a of the settling vessel 1534 may be selectively openable to facilitate flow of fluid from the settling vessel 1534 to the waste treatment system under gravity. In alternative arrangements, the fluid conduit 1562 may extend from the settling vessel 1534 toward or to the waste treatment system such that fluid falls therethrough under gravity. One or more valves 1540 may be provided along the fluid conduit 1542. The valve 1540 may be a solenoid valve. A flowmeter 1515 may be provided in the first fluid delivery means 1510, and/or may also be provided in the second fluid delivery means 1520.

The fluid delivery system 1510 is configured such that the treatment fluid is retained in the settling vessel 1534 for a period of time prior to delivery to the waste processing vessel 120. The period of time may be referred to as a settling time in some cases. The period of time may be referred to as a residence time of fluid in the settling vessel 1534. It will be understood that the period of time is a non-zero period of time. The period of time may be a predetermined settling time in some arrangements. It will be understood that the period of time relates to an elapsed time during which the treatment fluid is substantially stationary or not flowing through the fluid delivery system 1510 so as to allow the fluid to settle.

It will be understood that the settling vessel 1534 is substantially different to a pipe in which fluid may sit when valves are in a closed position. The settling vessel 1534 is configured to be substantially breathable or unsealed such that gas generated by or released from the treatment fluid can be removed from the settling vessel 1534. The settling vessel 1534 may be seen as a fluid well in which fluid can be held. The settling vessel 1534 is configured to isolate treatment fluid therein from the first fluid transfer means 1536 and the second fluid transfer 1538 means during settling. The settling vessel 1534 is configured to isolate treatment fluid therein from all conduits of the fluid delivery system 1510 during settling. The settling vessel 1534 is configured so that treatment fluid is delivered to the waste processing vessel 120 after the period of time.

The treatment fluid may include or be a gas-liquid mixture which can release or dissipate gas bubbles, which may be microbubbles. It will be understood that the dissipation of gas bubbles can increase the difficulty in measuring a true volume or volume flow rate of the treatment fluid delivered to the waste treatment system, for example using flowmeter 1515. Thus the presence of gas bubbles in the fluid may decrease the accuracy of control over the true volume of treatment fluid delivered to the waste treatment system. This can cause difficulty in ensuring an appropriate volume of treatment fluid is introduced into the waste treatment system. The configuration of the fluid delivery system 1510 allows the treatment fluid to release bubbles in the settling vessel 1534 prior to introduction of the fluid into the waste processing system. Agitation of the fluid and/or pressure variations created in the fluid as it is transferred through parts of the fluid delivery system, such as pipes, conduits, pumps or valves, can cause the fluid to generate gas bubbles, or 'fizz'. Such gas bubbles contribute to the overall volume of the fluid, but do not represent the true volume of active treatment fluid being delivered through the system. It is therefore proposed to allow these bubbles to settle out of the fluid before a determination of the volume of fluid being delivered to the waste to be treated is made. The settling vessel can provide a settling volume in which the fluid can rest relatively undisturbed, and its volume can then be measured at a point in the system where a sufficient amount of bubbles can be determined to have settled out of the fluid, so that the volume of the treatment fluid may be more accurately determined before delivery to the waste treatment system or its waste treatment vessel 120. It will be understood that the rate of release of bubbles of the treatment fluid may decrease with time the fluid is present in the settling vessel 1534. In this way, the treatment fluid may be more settled, or may release less bubbles, when it is transferred to the waste treatment system after settling in the settling vessel 1534 for a period of time, allowing for more precise control of the flow of treatment fluid and more effective and consistent treatment of waste in the waste treatment system. In this way, the period of time in which the treatment fluid is retained in the settling vessel 1534 can be understood to be the time required for an amount of gas bubbles in the fluid to reduce to a predetermined extent. In addition, settling the treatment fluid prior to introduction into the waste treatment system reduces the generation of bubbles in the waste treatment system, avoiding pressure increases and/or ineffective treatment of waste.

The fluid delivery system 1510 may include a sensor arrangement 1546. The sensor arrangement 1546 may be configured to measure a parameter associated with the fluid in the settling vessel 1534 and to generate a signal based on the parameter. The sensor arrangement 1546 can provide information regarding the state of the fluid in the settling vessel 1534. The sensor arrangement 1546 may generate a signal once the period of time has elapsed. In some arrangements, the sensor arrangement 1546 is configured to continuously measure a parameter associated with the fluid in the settling vessel 1534 and generate continuous or periodic signals based on this measurement. It should be understood that the sensor arrangement 1546 may include multiple sensors that measure various parameters associated with various components of the fluid delivery system 1510, including for example, pressure, temperature, flow of fluid into the settling vessel 1534, fluid level in the settling vessel 1534. The sensor arrangement 1546 may be coupled to a user output device, 600, for example a display for communicating the signal to operators.

In some arrangements, the fluid delivery system 1510 includes a controller 410 configured to control flow of treatment fluid into and/or out of the settling vessel 1534, for example, based on the signal from the sensor arrangement. The controller 1510 may be configured to control one or more valves of the first and/or second fluid transfer means 1536, 1538. As will be discussed in more detail in the "Control System" section, the controller 410 may include a memory 412 for storing the signal from the sensor arrangement 1546. The controller may be coupled to a user output device 600, e.g. a display for communicating various indicators to operators. It shall be appreciated that in alternative embodiments, any combination of these components may be present/omitted from the system.

The controller 410 is configured to determine whether a predetermined condition associated with the fluid in the settling vessel 1534 has been met based on the signal from the sensor arrangement 1546. The controller 410 is configured to generate a first output to instruct the second fluid transfer means 1538 to transfer the fluid from the settling vessel 1534 to the waste treatment system, e.g. to the waste processing vessel 120, based at least upon a determination that the first predetermined condition has been met. In this way, the fluid delivery system can effectively control the flow of treatment fluid between a fluid reservoir 1511 and waste treatment system via the settling vessel 1546.

The first output may be in the form of a signal, such as an electrical signal to the second fluid transfer means 1538. The signal is indicated in dashed lines in Figure 6A. In an example arrangement, the first output may be a signal, such as an electrical signal, from the controller 410 to the valve 1540 to move the valve 1540 from the closed position to the open position so as to allow treatment fluid to flow through the conduit 1542 to the waste treatment system (e.g. under gravity). The controller 410 may be configured to generate a further signal output to close the valve 1540 once the treatment fluid has been delivered from the settling vessel 1534 to the waste treatment system. This may facilitate further treatment fluid being introduced and retained in the settling vessel 1534. The controller 410 may be configured to determine that the treatment fluid has been delivered based on a signal from the sensor arrangement 1546. The controller 410 may be configured to generate the first output when the rate of generation of gas bubbles from the treatment fluid has decreased to a predetermined value. The predetermined condition determined by the controller 410 may be associated with the rate of generation of gas bubbles of the treatment fluid in the settling vessel 1534.

The predetermined condition may be or may include a minimum settling time over which the treatment fluid has been present in the settling vessel 1534. The controller 410 may be configured to determine whether the treatment fluid has been present in the settling vessel for a time greater than or equal to the minimum settling time and generate the first output in response thereto. The minimum settling time may be based on an amount of time taken for the rate of generation of gas bubbles from the treatment fluid to decrease below an acceptable value. The minimum settling time may be predetermined based on empirical or experimental data. The minimum settling time may be stored in the memory 412 of the controller 410. Configuring the controller 410 such that the treatment fluid is present in the settling vessel 1534 for a minimum settling time can allow a significant dissipation of gas bubbles to occur in the settling vessel, upstream of the waste treatment system, allowing for more effective control of the volume of the treatment fluid delivered to the waste treatment system.

The controller 410 may be configured to identify the presence of a treatment fluid in the settling vessel 1534 based on the signal from the sensor arrangement 1546. For example, the sensor arrangement 1546 may include one or more level sensors, optical sensors or the like. In some arrangements, the controller 410 may be configured to identify the time elapsed since instructing the introduction of treatment fluid into the settling vessel 1534 or since the end of the introduction of treatment fluid into the settling vessel 1534, or since identifying the presence of treatment fluid in the settling vessel 1534. The controller 410 may be configured to determine whether an elapsed time from identifying the presence of or instructing the introduction of treatment fluid in the settling vessel 1534 is greater than or equal to the minimum settling time so as to identify whether the predetermined condition has been met.

As noted above, the waste treatment system (e.g. the waste processing vessel 120) may be configured to perform one or more waste processing cycles. The or each waste processing cycle may operate for a predetermined cycle time. In some arrangements, the minimum settling time may be greater than or equal to the cycle time. The fluid delivery system 1510 may be configured to operate in parallel with the waste processing system. This allows the treatment fluid for a waste processing cycle to settle in the settling vessel 1534 while the previous cycle is ongoing.

In some arrangements, the controller 410 may be configured to generate the first output in response to a determination that the waste processing cycle is at the correct stage for the introduction of treatment fluid. For example, the waste processing cycle may include a fluid dispensing step in which treatment fluid in the waste processing vessel 120 is dispensed after the waste has been treated therein. The fluid dispensing step may be at the end of the waste processing cycle and may include the vessel 120 being rotated and emptied of processed waste and treatment fluid. The controller 410 may be configured to generate the first output in response to a determination that the fluid dispensing step has been reached and/or completed. In this way, fresh treatment fluid is only introduced into the waste processing system from the settling vessel 1534 when needed.

The predetermined condition may be or include detection or sensing of a volume of treatment fluid present in the settling vessel 1534. The predetermined condition may be or include a predetermined volume of treatment fluid present in the settling vessel 1534. In an example arrangement, the controller 410 may be configured to determine that a significant dissipation of gas bubbles from the treatment fluid has occurred in the settling vessel 1534 if the volume of treatment fluid in the settling vessel 1534 has reduced by a predetermined value. This value may be stored in the memory 412 of the controller 1534 (e.g. the value may be user-inputted). The predetermined condition may be or include a rate of change of volume of treatment fluid in the settling vessel 1534 with respect to time. It will be understood that the volume (e.g. the liquid volume) of treatment fluid in the settling vessel 1534 will decrease with time as gas bubbles are dissipated therefrom. The rate of generation of gas bubbles may decrease with time the treatment fluid is located in the settling vessel 1534. The rate of change of volume of treatment fluid in the settling vessel 1534 will decrease with time. In this way, the predetermined condition may be a maximum value for a rate of change of volume of the treatment fluid in the settling vessel 1534 with respect to time. The controller 410 may be configured to determine whether a rate of change of volume of the treatment fluid in the settling vessel is at or below the maximum rate of change based on the signal from the sensor arrangement 1546 and generate the first output in response thereto. The maximum rate of change of volume may be stored in the memory 412 of the controller 1534 (e.g. the value may be user-inputted). It will be understood that the term "rate of change of volume" refers to the rate of reduction in volume. The "maximum rate of change" may refer to the maximum rate of reduction in volume.

The controller 410 may be configured to generate the first output based upon a further or alternative determination that a predetermined volume of fluid is present in the settling vessel 1534. The predetermined volume of fluid may be based on the minimum volume of fluid required to be present in the waste treatment system. As noted above, the volume of treatment fluid in the settling vessel 1534 may decrease with time. Configuring the controller 410 in such a way can facilitate at least the predetermined volume of treatment fluid being present in the settling vessel 1534 before being introduced to the waste treatment system. Put another way, the controller 410 may be configured to generate the first output when the predetermined condition has been met and when the predetermined volume of treatment fluid is present.

The controller 410 may be configured to determine whether a volume of treatment fluid in the settling vessel 1534 is below the predetermined volume based on the signal from the sensor arrangement 1546 and in response thereto generate a second output to instruct the first fluid transfer means 1536 to deliver further treatment fluid into the settling vessel 1534 from the fluid reservoir 1511. In this way, the controller 410 is configured to compensate for a reduction in volume of the treatment fluid in the settling vessel 1534 (e.g. as a result of gas bubble dissipation) to ensure a desired volume of treatment fluid is introduced into the waste treatment system via the second fluid transfer means 1538.

The second output may be in the form of a signal, such as an electrical signal, to the first fluid transfer means 1536. In an example arrangement, the second output may be a signal, such as an electrical signal, from the controller 410 to the pump 1513 and/or valve 1514. The signal is indicated in dashed lines in Figure 6A. In an example arrangement, the valve 1514 may be configured to move from the closed position to the open position in response to the second output so as to allow treatment fluid to flow through the conduit 1512 from the reservoir 1511 to the settling vessel 1534. The pump 1513 may be configured to operate to draw fluid from the reservoir 1511 into the conduit 1512 and to the settling vessel 1534 in response to the second output. The controller 410 may be configured to generate a further output to close the valve 1513 and/or instruct the pump to stop drawing fluid from the reservoir 1511 once sufficient treatment fluid has been delivered from the reservoir 1511 to the settling vessel 1534. The controller 410 may be configured to determine that sufficient treatment fluid has been delivered based on a signal from the sensor arrangement 1546. In an example arrangement, the controller 410 may be configured to determine that the volume of treatment fluid in the settling vessel 1534 is at or above the predetermined volume based on the signal from the sensor arrangement 1546 and in response thereto generate an input to stop further treatment fluid being drawn into the settling vessel 1534 via the first fluid transfer means 1536.

The controller 410 may be configured to generate a third output to instruct the first fluid transfer means 1536 to draw a predetermined volume of treatment fluid from the fluid reservoir 1511 into the settling vessel 1534. The third output may be similar to the second output (e.g. an electrical signal that can control the pump 1513 and/or valve). The fluid delivery system 1510 can operate continuously or repeatably, allowing a batch of treatment fluid to settle in the setting vessel 1534 before being delivered to the waste treatment system for a waste processing cycle. The fluid delivery system 1510 can then introduce additional treatment fluid into the settling vessel 1534 to settle (via the third output) before being delivered to the waste treatment system for the next waste processing cycle. Configuring the controller 410 to generate the third output allows the fluid delivery system to operate automatically between multiple waste processing cycles (e.g. not requiring significant operator involvement). This can improve the efficiency of the overall waste treatment process.

The controller 410 may be configured to generate the third output after generating the first output. The controller 410 may be configured to detect that the treatment fluid has been transferred from the settling vessel 1534 to the waste processing vessel 120 (e.g. after generation of the first output) and in response thereto generate the third output. Such detection may be based on a signal from the sensor arrangement 1546. For example, the sensor arrangement 1546 may be configured to detect the presence of treatment fluid in the settling vessel 1534. When the sensor arrangement 1546 detects that the treatment fluid is not present in the settling vessel 1534, the controller 410 may be configured to determine that the treatment fluid has been transferred to the waste processing vessel 120. In other arrangements, the controller 410 may be configured to wait for a predetermined time after generating the first output before generating the third output.

The controller 410 may be configured to determine whether the fluid reservoir 1511 is empty, for example based on a signal from the sensor arrangement 1546) and in response thereto generate a fourth output. The fourth output may generate an alarm and/or message on a user output device 600, such as a display, to indicate to an operator that the reservoir 1511 has been depleted and requires refilling with treatment fluid. The controller 410 may determine whether the fluid reservoir 1511 is empty by comparing a volume of treatment fluid in the settling vessel 1534 with the predetermined volume. In an example arrangement, the controller 410 is configured to determine whether the volume of the treatment fluid in the settling vessel 1534 is less than the predetermined volume based on the signal from the sensor after generating the third output or the second output, and in response thereto generate a fourth output to indicate that the fluid reservoir is empty. It will be understood that the third output or second output are configured to instruct the first fluid transfer means 1536 to draw a volume of transfer fluid into the settling vessel 1534 such that the volume of fluid in the settling vessel 1534 is at or above the predetermined volume. The sensor arrangement 1546 may be configured to determine that the volume of transfer fluid in the settling vessel 1534 is below the predetermined volume, even after instructing the first fluid transfer means 1536. In this way, the controller 410 may be configured to determine that the reservoir 1511 is empty and/or there is a fault in the first fluid transfer means 1536 and generate a signal to alert an operator. Such an arrangement provides further automation of the fluid delivery system 1510, and prevents the system from incorrectly operating without transfer fluid.

The controller 410 may be configured to instruct the first fluid transfer means 1536 to deliver treatment fluid from the reservoir 1511 to the settling vessel 1534 upon determination that the waste treatment system is not performing a waste processing cycle, for example in between waste processing cycles or prior to performing a waste processing cycle. In some arrangements, treatment fluid may only be delivered to the settling vessel 1534 when the waste treatment system is not performing a waste processing cycle. It will be understood that a waste processing cycle may include significant movement and may agitate the treatment fluid being introduced into the settling vessel 1534. This may result in data from the sensor arrangement 1546 being inaccurate and may cause an imprecise introduction of treatment fluid into the waste treatment system. The controller 410 may be configured to generate the second output prior to the waste processing vessel performing a waste processing cycle. The controller may be configured to generate the third output prior to the waste processing vessel performing a waste processing cycle. In this way, upon determination that additional fluid is required in the settling vessel 1534, the controller 410 may be configured to first determine the status of the waste processing cycle before generating the second or third outputs.

The sensor arrangement 1546 may include one or more types of sensor, including for example flow sensors, volumetric sensors, level sensors, optical sensors, position sensors, or the like. The one or more sensors may be located within the settling vessel 1534 or external to the settling vessel 1534. The sensor arrangement may include one or more level sensors 1548. It will be appreciated that the level sensors 1548 provide a measurement associated with a volume of fluid in the settling vessel 1534. In some arrangements, the sensor arrangement 1546 includes a plurality of level sensors 1548. The parameter associated with the fluid in the settling vessel 1534 may be a volume or level of fluid (e.g. liquid) present in the settling vessel 1534. Level sensors can provide accurate and continuous measurements regarding the amount of treatment fluid in the settling vessel 1534, facilitating accurate and real-time information. Two level sensors 1548 are provided in the arrangement of Figure 6A, but it will be appreciated that any number or type of sensors may be provided. A plurality of level sensors 1548 advantageously provides for redundancy in case of failure of one of the sensors as well as increased accuracy and/or reliability of measurements. The one or more level sensors 1548 may be capacitive level sensors. It will be appreciated that the level sensors 1548 may be any suitable sensor, including conductive level sensors, float level sensors, optical level sensors, ultrasonic level sensors, laser level sensors, or the like.

The plurality of level sensors 1548 may be vertically aligned relative to each other with respect to a base region 1548a of the settling vessel 1534. The base region 1548a of the settling vessel 1534 defines a region in which liquid in the vessel 1534 will gather due to gravitational effects. The base region 1548a may be curved in some arrangements. Gas bubbles may dissipate from the treatment fluid in a direction away from the base region 1548a. The level sensors 1548 may be provided proximal a side wall of the settling vessel 1534. In some arrangements, the settling vessel 1534 may be at least partially formed from a transparent material, such as glass, such that the level sensors 1548 may be positioned external to the internal volume of the settling vessel 1534.

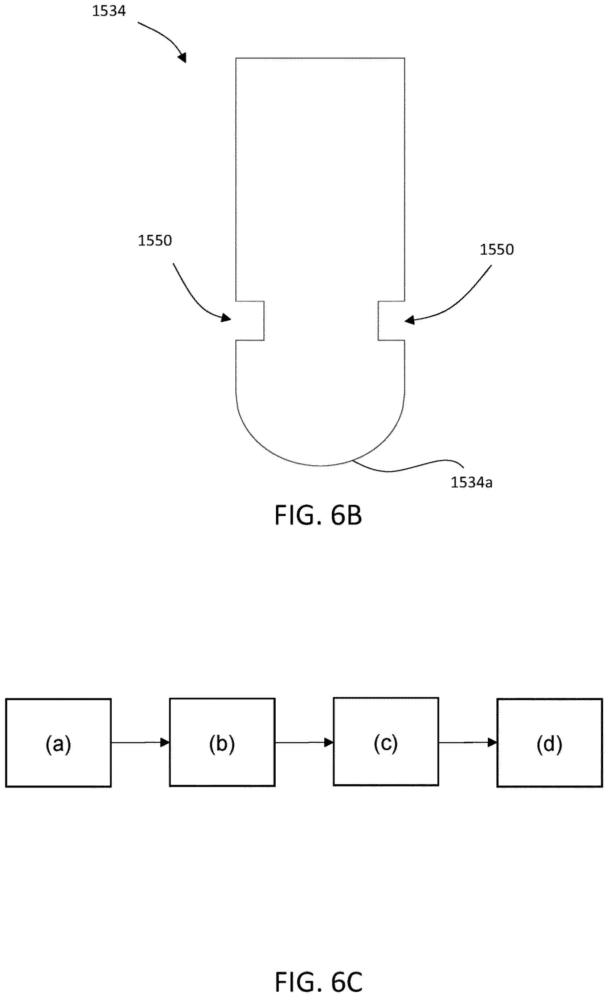

Figure 6B indicates an example arrangement of the settling vessel 1534. The settling vessel 1534 may include one or more formations for receiving or supporting a respective sensor 1548. In the illustrated arrangement, the formations are in the form of one or more slots 1550 in a wall of the settling vessel 1534. The or each slot 1550 is configured to receive a respective sensor, such as a respective level sensor 1548. A plurality of slots 1550, for example at least two, may be provided in a side wall of the vessel 1534, each slot 1550 is configured to receive a respective sensor. The slots 1550 are arranged such that the respective sensors 1548 are vertically aligned relative to each other with respect to the base region 1548a of the settling vessel 1534. The slots 1550 may be vertically and/or angularly aligned relative to each other. This improves the accuracy of measurement from the sensor arrangement 1546 and therefore improves the precision of the fluid delivery system 1510. In some arrangements, a single continuous slot may extend around the side wall of the settling vessel 1534 so as to allow the level sensors 1548 to be vertically aligned in any position about the wall. In alternative arrangements, one or more of the formations may be in the form of a projection or shoulder extending from a wall of the settling vessel 1534, upon which a sensor 1548 can be supported.

The sensors 1548 of the sensor arrangement 1546 may be configured to measure the parameter associated with the fluid at predetermined periods during operation of the waste processing system. In some arrangements, the sensors 1548 are configured to measure the parameter continuously or periodically. In this case, the sensor arrangement 1546 may be configured to provide the signal to the controller 410 based on the parameter at predetermined periods during operation of the waste processing system. In other arrangements, the sensor arrangement 1546 may send continuous or periodic signals to the controller 410. The controller 410 may be configured to only consider the signal at predetermined periods during operation of the waste processing system. As has been previously noted, movement of component parts of the waste processing system (e.g. blade arrangement) during the waste processing cycle may agitate the treatment fluid in the settling vessel 1534, reducing the accuracy of readings from the sensor arrangement 1546. The controller 410 may be configured to only consider and/or receive signals from the sensor arrangement 1546 after completion of a waste processing cycle or during downtime of a cycle, for example when component parts are stationary. This improves accuracy of readings from the sensor arrangement 1546 and thus improves the precision of the fluid delivery system 1510.

The fluid delivery system 1510 may be a first fluid delivery system. The fluid delivery means 100 may include a second fluid delivery system 1520. A third fluid delivery system 1530 may be included. The second fluid delivery system 1520 may be for delivering a diluent to the waste processing vessel 120. The third fluid delivery system 1530 may be for delivering a neutralising agent to the waste processing vessel 120. As used herein, a "diluent" relates to a substance that can have a diluting effect on the treatment fluid. The diluent may comprise, consist essentially of, or preferably consist of water. The second fluid delivery system 1520 and/or third fluid delivery system 1530 may include any of the features of the first fluid delivery system 1510 in any combination. It will be appreciated that the second and/or third fluid delivery systems 1520, 1530 may include any or all of the features of the first fluid delivery system 1510. In example arrangements, the second and/or third fluid delivery systems 1520, 1530 may include a settling vessel along the fluid line, such that the diluent and/or neutralising agent can settle prior to delivery to the waste processing vessel 120.

The first and/or second fluid delivery means can deliver treatment fluid and/or diluent to the closure arrangement, such as lid 170, which is arranged to close the waste processing vessel 120. This delivery may occur via a spray nozzle 1552 provided on an inside surface of the lid 170. The second fluid delivery means 1520 may comprise an inlet 1522. The inlet 1522 may be configured for connection to a diluent source. The diluent source may be from a container, or may be from an external water supply such as a mains water supply. The inlet 1522 may pass via a switching means such as the valve 1514 or a further separate valve provided for this function, and may be subsequently connected to the closure arrangement such as lid 170, to deliver diluent into the waste processing vessel 120. The third fluid delivery means 1530 may be configured similarly to the first or second fluid delivery means, having features equivalent to one or more, or all of, the features of the first or second fluid delivery means 1510. In particular, a flowmeter may be provided in the third fluid delivery means 1530. The third fluid delivery means may deliver a fluid provided in a container 1531, similarly configured to the container 1511 of figure 5A. The container 1531 is represented schematically by dashed lines in figure 5A to enable features behind it in that view to be seen. Fluid may be delivered from the container 1531 via a fluid delivery conduit 1532. The fluid delivery conduit 1532 may pass via a pumping means 1533. The pumping means 1533 may have similar, or same features, as those of the pumping means 1513. Fluid delivery conduit 1532 is connected to the closure arrangement 172 deliver fluid therethrough. Passing via the lid is of course optional and the treatment fluid and/or diluent could be delivered to the waste processing vessel by other paths, such as through walls or a base of the vessel. While one means of delivering treatment fluid, treatment agents, or neutralisation fluids or agents as disclosed herein to the vessel 120 is via the described fluid delivery means, it will be understood that treatment agents can be provided in any suitable manner, in liquid, gel, solid or powdered form.

It may be the case that the temperature of the diluent is in the range of from about 1 °C to about 40 °C. It may be the case that the temperature of the diluent is in the range of from about 10 °C to about 30 °C.

Referring to Figure 6C, a method of delivering a treatment fluid to a waste treatment system is indicated. The method may include any or all of the following steps:

    (a) transferring a treatment fluid from a fluid reservoir 1511 to a settling vessel 1534 configured to retain the fluid;
    (b) retaining the treatment fluid in the settling vessel 1534 for a period of time; and
    (c) transferring the treatment fluid from the settling vessel to a waste treatment system after the period of time has elapsed.

The method may further include any or all of the below optional steps:

    (d) measuring a parameter associated with the fluid in the settling vessel 1534;
    (e) determining whether a predetermined condition associated with the fluid in the settling vessel 1534 has been met based on the measured parameter; and
    (f) transferring the treatment fluid from the settling vessel 1534 to a waste treatment system when it is determined that the predetermined condition has been met.

One or more of the method steps above may be performed by a controller or manually by an operator. It will be understood that although the fluid delivery system 1510 and method have been described as delivering a treatment fluid to the waste delivery apparatus 1000 described herein, the fluid delivery system and method may be utilised in delivering a treatment fluid to any waste treatment system, for example any biohazardous waste treatment system.

**Operating Sequence**

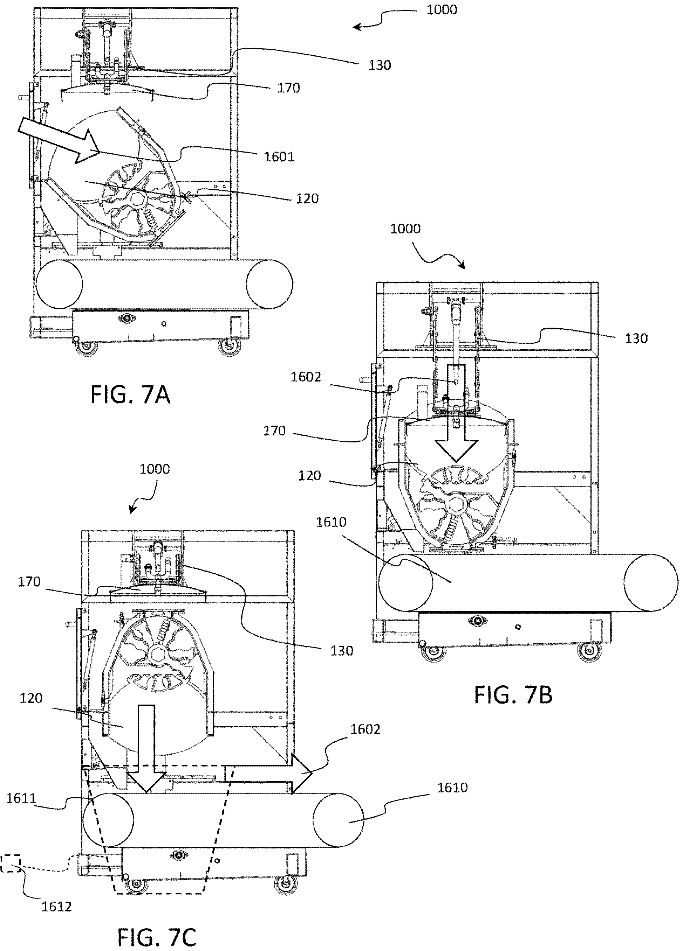

Operation of the waste processing apparatus will now be described with reference to Figures 7A to 7C. As will be understood, the waste processing vessel can be arranged in a plurality of different positions for carrying out different stages of the operation. A first position of the waste processing vessel may be a waste loading position. An example of such a waste loading position is shown in Figure 7A. In the waste loading position, the closure arrangement 170 is in an open configuration. Closure arrangement 170 may be mounted to the chassis or enclosure within which the waste processing vessel 120 is disposed. The closure arrangement 170 may therefore not be directly connected to the waste processing vessel. In the example illustrated, the closure arrangement 170 is therefore retracted from and distanced from the waste processing vessel.

An operating sequence may start by orienting the waste processing vessel 120 in the first position, in which waste may be loaded into the waste processing vessel 120. This may include orienting the waste receiving opening 1270 of the waste processing vessel 120 towards a loading position, and may include arranging the waste receiving opening 1270 towards the loading hatch 160 to enable waste to be loaded through the loading hatch 160 into the waste processing vessel 120 in the direction of arrow 1601 as shown in Figure 7A.

The waste processing vessel 120 may be moved to a second position. The second position can involve orienting the waste receiving opening 1270 towards a more upward or more vertical orientation relative to the first position. An example of such a second position is shown in Figure 7B. The waste receiving opening is oriented upwardly so that fluids added to the vessel remain in the vessel due to gravity and do not tend to pour or spill from the vessel 120. In this orientation, the closure arrangement 170 may be advanced towards the waste processing vessel 120 to close or substantially close the waste receiving opening, such as by advancing the closure arrangement 170 in the direction of arrow 1602 in Figure 7B. This may help prevent waste and/or treatment fluid and/or diluent and/or vapour from escaping from the waste processing vessel 120. An amount of treatment fluid and/or diluent may then be added to the waste processing vessel via first 1510 and/or second 1520 fluid delivery systems. With the waste processing vessel in a closed configuration, the rotatable blade array is rotated so as to carry out a combined shredding and mixing operation, to shred waste between the rotatable blade array and the fixed blade array(s) and also to mix the waste with the treatment fluid in the bottom of the waste processing vessel 120.

The apparatus can advantageously shred waste to a sufficiently small size that the waste can be immersed in treatment fluid in the waste processing vessel 120, which may take place in the waste processing zone Z. This can be performed for all waste added to the waste processing vessel. One way of achieving this is for the waste to be shredded to a size smaller than a depth of the treatment fluid retained the waste processing vessel, for example so that it fits below level S. The apparatus can also advantageously mix the waste with treatment fluid during and/or after the shredding of the waste by the blades. This can be achieved by continued operation of the blades for a sustained period. An advantage of methods described herein is that no heat energy need necessarily be applied to the waste for the treatment process. This can provide economies in energy consumption.

The arrangement of the blade arrangement in the waste processing vessel is such that it can shred the waste into smaller particles and provide a number of advantageous functions and advantages. These can include exposing the material to the chemical for disinfection, making the waste unrecognisable, for example so that the form or function of the un-shredded waste is unrecognisable, or such that any branding or personal data applied to the waste is unrecognisable. This can be advantageous when handling medical waste which may have this type of data applied. A further function may be to render the waste un-reusable, so that it can no longer perform the function it was manufactured for, for example such that a syringe or sample tube could not be re-used for the same function. It is also relevant that the entire treatment vessel is disinfecting waste regardless of whether or not it is in the liquid area.

An advantage of the system described herein is that both the functions of shredding and of disinfecting are carried out in the same enclosed area. This can be advantageous as compared to systems which may shred waste in a first area or operation and then treat, sterilise or disinfect the waste in a second area or operation. If the functions are separated, in the intermediate phase between shredding and disinfection, there could be a risk of aerosol infection, in particular if intervention for maintenance, for example, is required when a waste processing cycle is incomplete.

An aspect of the apparatus described is that it can apply the treatment fluid from the top down, which may be carried out via the first 1510 and/or second 1520 fluid delivery systems. If performed before the blades are rotated, this can help to provide that the waste is substantially covered in treatment fluid prior to commencing shredding. Subsequently, the shred process may start by rotating the blades at a first speed, which may be a lower speed, while a higher rotation speed may be used later in the cycle. Treatment fluid or diluent may be applied simultaneously with rotation of the blades, at any point during the shredding process, and in particular at the beginning of the shredding process. Applying fluid from the top down during shredding can also help to prevent infectious aerosols propagating from the waste away from the treatment fluid, for example in an upward direction. This risk can be greatest at the beginning of the cycle, when containers are initially broken down and opened, so applying a spray at the early stages can have a particular advantage.

When the waste processing operation is complete, the closure mechanism 130 may be actuated to open the closure arrangement 170 and the waste processing vessel 120 may be moved to a third position, in which the opening 1270 faces at least partially, or fully, downwards. In this orientation, waste and/or treatment fluid may be tipped out of the waste processing vessel 120 through the opening 1270. An example of such a third position is shown in Figure 7C. This tipping action may deliver the waste and/or treatment fluid to a conveying means. Such conveying means may include a linear conveyor 1610, or could alternatively be a rotational conveying means such as an auger. Fluid may be able to drain from the waste onto, and drain off or out of, the conveying means. The waste may then be conveyed, such as in the direction of arrow 1603 in Figure 7C, for discharge from the waste processing apparatus 1000. The waste may be delivered to a further conveying means, for example, for conveying to a further location, such as in an upward direction, to allow the waste to be delivered to a receiving vessel such as a waste bin or other container. If a shredding process is aborted, the waste may instead be ejected into a reject bin as shown by dashed lines 1611, which may carry a reject bin identifier plug 1612, for plugging into a reject bin identifier socket, to indicate to the controller 410 of the apparatus 1000 that the reject bin 1611 is in place.

**Chemical Process**

The treatment fluid may comprise liquid, such as water, and may comprise a treatment agent. The treatment fluid may be in the form of a homogenous liquid mixture or a solution. The treatment agent may be provided in solid, liquid or gel form and may be combined with a diluent, which may be a liquid diluent such as water. As described herein, the term "diluent" takes its usual meaning in the art and relates to a substance that is added to dilute a solution or a mixture. The treatment fluid may comprise a disinfectant. The treatment fluid may comprise a disinfectant and a diluent. The treatment fluid may comprise a solution comprising a disinfectant and a diluent. The treatment fluid may be acidic. The pH of the treatment fluid may be in the range of from about 2 to about 6.

As used herein, the term "solution" relates to liquid mixtures in which the minor component(s) (i.e., the disinfectant) is uniformly distributed throughout the major component (i.e., a diluent as described above with respect to the waste processing apparatus). The diluent may comprise, consist essentially of or preferably consist of water. The solution as described herein may also be referred to as the "disinfectant solution". The disinfectant solution may be acidic. The pH of disinfectant solution may be in the range of from about 2 to about 6.

The disinfectant solution may be provided via the first 1510 and/or second 1520 fluid delivery systems. It may be the case that at least some of the total volume of the diluent is provided to the vessel 120 via the second fluid delivery means. The disinfectant solution may therefore be provided in a 'pre-mixed' state via only one fluid delivery means into the waste processing vessel 120. Alternatively, different components of the disinfectant solution may be delivered separately into the waste processing vessel via different fluid delivery means. The diluent may comprise, consist essentially of or preferably consist of water.

In one example, in a first step, a first component, such as the disinfectant or treatment fluid, is provided to the waste processing vessel 120 via the first fluid delivery system 1510 in a desired amount. A second component such as the diluent may be delivered to the waste processing vessel in a second amount (e.g. via the second fluid delivery system 1520), to achieve a desired mixture, with desired relative concentrations, of the first and second components. In a first example, the diluent and disinfectant or other component(s) are added to the vessel simultaneously. However, it will be appreciated that in other examples, the diluent may be added to the waste processing vessel in a first step and followed by the disinfectant or other component, or vice versa. Adding the diluent first may avoid high concentrations of disinfectant in the vessel 120, for example.

As used herein, the term "disinfectant" relates to a chemical agent, which is able to inactivate viruses, bacteria, and other microorganisms that can cause infection and disease. The term "treatment fluid" may also be used.

The disinfectant may comprise peracetic acid and hydrogen peroxide. As used herein, peracetic acid may also be referred to as peroxyacetic acid.

Without being bound by theory, peracetic acid and hydrogen peroxide demonstrate a synergistic effect when used in combination against infectious agents (such as microorganisms) present in biohazardous waste. For example, hydrogen peroxide can erode the outer layer of the microorganism, allowing peracetic acid to more efficiently degrade the proteins and nucleic acids contained within.

A combination of peracetic acid and hydrogen peroxide results in an equilibrium reaction as outlined below in Equation (I) below:

$$\text{Equation (I): } CH_3C(=O)OH + H_2O_2 \rightleftharpoons CH_3C(=O)OOH + H_2O$$

The term "equilibrium reaction" takes its usual meaning and relates to a chemical reaction in which two opposing chemical reactions are occurring simultaneously. The equilibrium may be shifted by changing various parameters, such as the reaction temperature, pressure, incorporation of a catalyst, or the composition of the reactants/products.

In Equation (I) Acetic acid and hydrogen peroxide react to generate peracetic acid and water. Peracetic acid in the presence of water will degrade to hydrogen peroxide and acetic acid over time.

Peracetic acid may be present in the range of from about 10% to about 20% based on the total weight of the disinfectant. It may be the case that peracetic acid is present in the range of from about 12% to about 17% based on the total weight of the disinfectant. At least 13% peracetic acid may be present based on the total weight of the disinfectant. At least 14% peracetic acid may be present based on the total weight of the disinfectant. At least 15% peracetic acid may be present based on the total weight of the disinfectant. At least 16% peracetic acid may be present based on the total weight of the disinfectant.

Hydrogen peroxide may be present in the range of from about 15% to about 30% based on the total weight of the disinfectant. It may be the case that hydrogen peroxide is present in the range of from about 18% to about 25% based on the total weight of the disinfectant. At least 20% hydrogen peroxide may be present based on the total weight of the disinfectant. At least 21% hydrogen peroxide may be present based on the total weight of the disinfectant. At least 22% hydrogen peroxide may be present based on the total weight of the disinfectant. At least 23% hydrogen peroxide may be present based on the total weight of the disinfectant.

Peracetic acid and hydrogen peroxide present at the abovementioned weight percentages drives the equilibrium reaction shown in Equation (I) towards the formation of peracetic acid and water, without the need for the addition of a strong acid catalyst, such as sulfuric acid. It is desirable to avoid the need for addition of strong acid catalysts, as they are corrosive and difficult to handle.

Moreover, without being bound by theory, driving the equilibrium reaction towards the formation of peracetic acid provides greater disinfectant efficacy in a shorter space of time compared to known disinfectants. For instance, the disinfectant according to the disclosure gives rise to a log reduction over a short cycle time. As used herein, the term "log reduction" takes its usual meaning in the art and relates to the mathematical term used to express the relative number of microorganisms (such as bacteria and viruses, or bacterial or fungal spores) that are inactivated by a disinfectant. The Log reduction value indicates the percentage kill rate of microorganisms. Log reduction can be measured using any known technique. For instance, a biological indicator as described herein below can be exposed to the treatment fluid from the waste processing zone of the waste processing vessel during a waste processing cycle of the waste processing apparatus. The biological indicator can then be extracted following completion of the processing cycle and placed in a culture medium and incubated for a pre-determined time (depending on the nature of the biological indicator in question and its respective desired culture conditions). Following incubation, absence of growth of microorganisms in the culture medium may indicate an efficient disinfection process. For example, an absence of growth may indicate a 99.9999% reduction in the number of active microorganisms and/or a log reduction in the number of active microorganisms. In instances where the biological indicator comprises about $10^4$ microorganisms prior to the treatment process, a log 4 reduction following treatment would be indicative of an efficient disinfection process. In instances where the biological indicator comprises about $10^5$ microorganisms prior to the treatment process, a log 5 reduction following treatment would be indicative of an efficient disinfection process. In instances where the biological indicator comprises about $10^6$ microorganisms prior to the treatment process, a log 6 reduction following treatment would be indicative of an efficient disinfection process. In instances where the biological indicator comprises about $10^7$ microorganisms prior to the treatment process, a log 7 reduction following treatment would be indicative of an efficient disinfection process. In instances where the biological indicator comprises about $10^8$ microorganisms prior to the treatment process, a log 8 reduction following treatment would be indicative of an efficient disinfection process. In instances where the biological indicator comprises about $10^9$ microorganisms prior to the treatment process, a log 9 reduction following treatment would be indicative of an efficient disinfection process. In instances where the biological indicator comprises about $10^{10}$ microorganisms prior to the treatment process, a log 10 reduction following treatment would be indicative of an efficient disinfection process.

The cycle time for achieving a log reduction during the waste treatment process may be 20 minutes or less, 18 minutes or less, 16 minutes or less, 14 minutes or less, 12 minutes or less, 11 minutes or less, 10 minutes or less, 9 minutes or less, 8 minutes or less, 7 minutes or less, 6 minutes or less, 5 minutes or less, 4 minutes or less, or 3 minutes or less. In certain examples, the cycle time may be as little as 2 minutes. Factors influencing the effectiveness of different cycle times include the efficiency of the shredding arrangement in shredding solid materials and/or closed containers, so that contaminated surfaces are exposed to the treatment fluid, combined with the chemical efficacy of the treatment fluid comprising a solution comprising a disinfectant and a diluent (also referred to as the disinfectant solution). Therefore, particularly short cycle times may be effective when using the treatment fluids described herein, or the shredding arrangements described herein, and particularly when using those in combination.

It may be the case that the disinfectant solution comprising a diluent and a disinfectant comprising peracetic acid and hydrogen peroxide is of a concentration in the range of from about 0.5% (v/v) to about 5% (v/v). It may be the case that the disinfectant solution comprising a diluent and a disinfectant comprising peracetic acid and hydrogen peroxide is of a concentration in the range of from about 1% (v/v) to about 3% (v/v). It may be the case that the concentration is at least 1% (v/v). It may be the case that the concentration is at least 2% (v/v). It may be the case that the concentration is at least 3% (v/v). As used herein, the term "v/v" denotes volume per volume for concentration of a solution wherein the components within the solution are liquid. The diluent may comprise, consist essentially of or preferably consist of, water.

It may be the case that the disinfectant comprising peracetic acid and hydrogen peroxide is present in the range of from about 100 millilitres to about 1000 millilitres. It may be the case that the disinfectant comprising peracetic acid and hydrogen peroxide is present in the range of from about 300 millilitres to about 800 millilitres. It may be the case that at least 600 millilitres of disinfectant comprising peracetic acid and hydrogen peroxide is present. It may be the case that at least 700 millilitres of disinfectant comprising peracetic acid and hydrogen peroxide is present.

It may be the case that the diluent is present in the range of from about 10 litres to about 30 litres. At least 15 litres of diluent may be present. At least 20 litres of diluent may be present. At least 25 litres of diluent may be present.

It may be the case that the disinfectant solution comprises a diluent and a disinfectant comprising peracetic acid in the range of from about 10% to about 20% based on the total weight of the disinfectant and hydrogen peroxide in the range of from about 15% to about 30% based on the total weight of the disinfectant; and wherein the solution is of a concentration in the range of from about 0.5% (v/v) to about 5% (v/v). The diluent may comprise, consist essentially of or preferably consist of water.

It may be the case that the solution comprises a disinfectant and a diluent, wherein the disinfectant comprises peracetic acid in the range of from about 12% to about 17% based on the total weight of the disinfectant and hydrogen peroxide in the range of from 18% to 25% based on the total weight of the disinfectant; and wherein the solution is of a concentration in the range of from about 1% (v/v) to about 3% (v/v). The diluent may comprise, consist essentially of or preferably consist of water.

It may be the case that the disinfectant solution further comprises an antifoaming agent. The antifoaming agent may be a silicone-based antifoaming agent, a non-silicone based antifoaming agent, or a combination thereof.

As used herein, the term "antifoaming agent" takes its usual meaning in the art and relates to chemical agents which can control the formation of foam.

A silicone based antifoaming agent relates to antifoaming agents comprising polymers with silicon backbones that are either oil or water based. Non-silicone-based antifoaming agents include mineral and organic based products, such as hydrocarbon based antifoaming agents.

Examples of silicone-based antifoaming agents include, but are not limited to, organopolysiloxane oils, such as poly(dimethylsiloxane), poly(methylphenylsiloxane), poly(methylethylsiloxane), poly(diethylsiloxane), poly (ethylphenylsiloxane), or combinations thereof.

Examples of non-silicone based antifoaming agents include, but are not limited to, hydrocarbon-based antifoaming agents. For example, hydrocarbon based antifoaming agents may comprise an aliphatic hydrocarbon oil, an aromatic hydrocarbon oil, an acyclic hydrocarbon oil, or combinations thereof. Additionally, or alternatively, the antifoaming agent may be selected from the foam control agents in the Foamdoctor range from PennWhite® Global chemical solutions (e.g., FoamDoctor® G2777E, FoamDoctor® G2005, FoamDoctor® G2020, FoamDoctor® G2030, or Foam-Doctor® G2898).

The antifoaming agent may be present at a volume in the range of from about 5 millilitres to about 50 millilitres. It may be the case that the antifoaming agent is present at a volume of from about 10 millilitres to about 30 millilitres. It may be the case that at least 50 millilitres of antifoaming agent are present. It may be the case that at least 40 millilitres of antifoaming agent are present. It may be the case that at least 30 millilitres of antifoaming agent is present. It may be the case that at least 20 millilitres of antifoaming agent is present.

The antifoaming agent may be provided together with the disinfectant solution. It may be the case that the antifoaming agent is provided via the first 1510 and/or second 1520 fluid delivery means together with the disinfectant and/or diluent. It may be the case that at least some of the total volume of the antifoaming agent is provided to the vessel 120 via the second fluid delivery means.

The antifoaming agent may be added to the waste processing vessel at the same time as the biohazardous waste to be treated. It may be the case that the antifoaming agent is housed within a container. The container may be flexible or solid. The container may be tubular, conical, cubic, or have a bottle-like shape with a neck. and may be substantially cylindrical or may be a sealed bag. The container may comprise a material which is compatible with and does not influence the liquid treatment process. The container may comprise glass, glass fibers, plastic, ceramics, stainless steel, and metal oxides. It may be the case that the container comprises plastic.

Addition of the antifoaming agent in a container as described herein delays contact of the antifoaming agent contained within the biohazardous waste and treatment fluid, as the antifoaming agent is only released upon shredding of the container. Upon simultaneous shredding and treatment of the biohazardous waste, the level of foaming is often more pronounced. Therefore, addition of an antifoaming agent in a container will allow for optimum defoaming of the waste material and treatment fluid within the waste processing vessel.

The disinfectant solution may further comprise a fragrance additive. As used herein, the term "fragrance additive" takes its usual meaning in the art and relates to fragrance additives that are used to mask any unwanted odours of the treatment fluid. The fragrance additive may comprise an aldehyde (such as an aromatic aldehyde or fatty aldehyde comprising between 8 and 13 carbon atoms), a ketone, an ester (such as an aliphatic ester), an ether, an acetate, a nitrile, a terpene hydrocarbon, or an essential oil (such as natural essential oils or synthetic essential oils). An example of an aromatic aldehyde includes, but is not limited to, cinnamic aldehyde. It may be the case that the fragrance additive is RESAFRESH RIO® by MACNAB SALES.

The fragrance additive may be present at a volume in the range of from about 5 millilitres to about 50 millilitres. It may be the case that the antifoaming agent is present at a volume of from about 10 millilitres to about 30 millilitres.

According to another aspect of the disclosure, there is provided a method of processing waste, the method comprising the following steps:

(I) addition of waste and a treatment fluid to a waste processing vessel of a waste processing apparatus, wherein the waste processing vessel comprises a waste processing zone, and wherein the treatment fluid is retained in the waste processing zone; and
(II) activation of a blade arrangement, such that the waste in the waste processing vessel can be shredded by the blade arrangement while immersed in the treatment fluid retained in the waste processing zone of the waste processing vessel; and
(III) deactivation of the blade arrangement after a pre-determined cycle time.

The waste, treatment fluid, and waste processing apparatus can be provided as described hereinabove.

As used herein, the cycle time relates to the time elapsed between the beginning of the mechanical processing, or shredding, of the waste in the presence of the treatment fluid. It therefore begins when the waste and the treatment fluid are present in the waste processing vessel together, and the blade arrangement begins shredding of the waste. It ends when the shredding and mixing of the waste in the vessel 120 stops. When the treatment fluid comprises a disinfectant solution as described herein, the cycle time may be the time taken, or the time sufficient, to reach a log reduction.

The method for treating waste may take place at a temperature in the range of from about 1 °C to about 40 °C, optionally in the range of from about 5 °C to about 30 °C, optionally in the range of from about 10 °C to about 26 °C.

According to another aspect of the disclosure, there is provided the use of a solution comprising a disinfectant and a diluent (also referred to as a disinfectant solution) in the treatment of biohazardous waste. The disinfectant solution may be acidic. The pH of the disinfectant solution may be in the range of from about 2 to about 6.

The disinfectant may comprise peracetic acid and hydrogen peroxide. The diluent may comprise, consist essentially of or preferably consist of water. The disinfectant may comprise peracetic acid in the range of from about 10% to about 20% based on the total weight of the disinfectant. The disinfectant may comprise peracetic acid in the range of from about 12% to about 17% based on the total weight of the disinfectant.

The disinfectant may comprise hydrogen peroxide in the range of from about 15% to about 30% based on the total weight of the disinfectant. The disinfectant may comprise hydrogen peroxide in the range of from about 18% to about 25% based on the total weight of the disinfectant. The solution may be of a concentration in the range of from about 0.5% (v/v) to about 5% (v/v). The solution may be of a concentration in the range of from about 1% (v/v) to about 3% (v/v).

According to another aspect of the disclosure, there is provided a use of a solution comprising a disinfectant and a diluent comprising water in the treatment of biohazardous waste, wherein the disinfectant comprises peracetic acid in the range of from about 10% to about 20% based on the total weight of the disinfectant and hydrogen peroxide in the range of from about 15% to about 30% based on the total weight of the disinfectant.

According to another aspect of the disclosure, there is provided a use of a solution comprising a disinfectant and a diluent comprising water in the treatment of biohazardous waste, wherein the disinfectant comprises peracetic acid in the range of from about 10% to about 20% based on the total weight of the disinfectant and hydrogen peroxide in the range of from about 15% to about 30% based on the total weight of the disinfectant; and wherein the solution is of a concentration in the range of from about 0.5% (v/v) to about 5% (v/v).

According to another aspect of the disclosure, there is provided a use of a solution comprising a disinfectant and a diluent comprising water in the treatment of biohazardous waste, wherein the disinfectant comprises peracetic acid in the range of from about 12% to about 17% based on the total weight of the disinfectant and hydrogen peroxide in the range of from about 18% to about 25% based on the total weight of the disinfectant, and wherein the solution is of a concentration in the range of from about 1% (v/v) to about 3% (v/v), in the treatment of biohazardous waste.

It is desirable to disinfect biohazardous waste, such that it can be disposed of safely or recycled. Moreover, there is a desire to disinfect biohazardous waste as quickly as possible. Use of a solution comprising a disinfectant comprising peracetic acid and hydrogen peroxide in the treatment of biohazardous waste as described above provides for effective chemical treatment of biohazardous waste, wherein a log reduction is achieved over a short cycle time. Moreover, use of a disinfectant solution described herein in combination with aspects of the waste processing apparatus described herein, whereby waste being shredded is immersed in a disinfectant solution during shredding, not only results in a log reduction over a short cycle time, but overcomes the setbacks associated with prior art systems in which, in order for repeated passes through a standard vertical shredding device to take place, waste must be drawn out of treatment fluids and re-passed vertically through a shredder arrangement, such as a conventional two-shaft shredding arrangement in which waste passes vertically downwards through and between the shafts and their associated shredding blades such that a disinfectant solution would generally run off the waste being shredded and treated while it passes once more through the shredding arrangement.

**Post-processing Neutralisation**

The method of treating waste described herein above may further comprise an additional step of adding a neutralising agent (also referred to as neutralisation). As used herein, the term "neutralisation" or "neutralisation process" takes its usual meaning in the art and relates to the chemical reaction involving an acid and a base to form a salt and water and indicates a state of equilibrium between acidity and alkalinity. A neutralisation reaction will typically have a neutral pH (i.e. proximate to a pH of 7). The exact pH value will be dependent on the temperature of the solution in question.

The additional step of adding a neutralising agent may follow deactivation of the blade arrangement after the predetermined cycle time. It may be the case that the waste and treatment fluid are neutralised prior to emptying the waste processing vessel. It may be the case that the treatment fluid is neutralised once it has been emptied from the waste processing vessel.

Neutralisation may comprise addition of a neutralising agent. After completion of the waste processing operation, the treatment fluid is not always suitable for direct discharge into a municipal sewer. For instance, where the treatment fluid comprises a solution comprising a disinfectant and a diluent as described herein (also referred to herein as a disinfectant solution), the pH may be in the range of from about 2 to about 6. As such, it may be too acidic for direct discharge into a municipal sewer, risking corrosion of the sewer walls, and the production of unwanted gaseous by-products within the sewer.

As used herein, the term "neutralising agent" relates to a chemical agent that is capable of adjusting the pH of the treatment fluid as described herein towards a neutral pH. It may be the case that the neutralisation agent is capable of adjusting the pH of the treatment fluid to a pH in the range of from about 6 to about 10, often about 6 to about 9.5, often about 6 to about 8, often about 6.5 to about 7.5. The pH of the treatment fluid before neutralisation with the neutralising agent may be in the range of from about 2 to about 6. The pH of the treatment fluid before neutralisation with the neutralising agent may be in the range of from about 2 to about 4. Where the treatment fluid is acidic, for example when peracetic acid is used as part of the liquid treatment process, then the neutralising agent serves to increase the pH of the acidic treatment fluid.

The pH of the treatment fluid following neutralisation with the neutralising agent as defined herein may be in the range of from about 4 to about 10. It may be the case that the pH of the treatment fluid following neutralisation with the neutralising agent is in the range of from about 6 to about 10. It may be the case that the pH of the treatment fluid following neutralisation with the neutralising agent is in the range of from about 6 to about 9.5. It may be the case that the pH of the treatment fluid following neutralisation with the neutralising agent as defined herein is in the range of from about 6 to about 8. It may be the case that the pH of the treatment fluid following neutralisation with the neutralising agent as defined herein is in the range of from about 6.5 to about 7.5. It may be the case that the pH of the treatment fluid following neutralisation with the neutralising agent as defined herein is proximate to 7. Such pH values allow for the treatment fluid to be directly discharged into a municipal sewer, minimising the risk of corrosion of the sewer walls, and the production of unwanted gaseous by-products within the sewer.

The pH of the neutralising agent may be in the range of from about 9 to about 14. The neutralising agent may comprise a strong base, a weak base, or a combination thereof.

The neutralising agent may comprise a strong base. As used herein, the term "strong base" takes its usual meaning in the art and relates to a base that is completely dissociated in an aqueous solution. The strong base may comprise a group I metal hydroxide, a group II metal hydroxide, or combinations thereof. The strong base may comprise lithium hydroxide, sodium hydroxide, potassium hydroxide, calcium hydroxide, rubidium hydroxide, strontium hydroxide, cesium hydroxide, barium hydroxide, or a combination thereof. The strong base may comprise sodium hydroxide, potassium hydroxide, calcium hydroxide, or a combination thereof. The strong base may comprise sodium hydroxide. Strong bases as described herein provide for effective and rapid neutralisation of an acidic treatment fluid (as those described herein), resulting in a treatment fluid that is suitable for immediate discharge directly to a municipal sewer. In addition, strong acids will nullify the biocidal effect of the treatment fluid described herein. Eliminating the biocidal effect of the treatment fluid prevents any detrimental impact to the environment, and the production of unwanted gaseous by-products within the sewer.

The neutralising agent may comprise a weak base. As used herein, the term "weak base" takes its usual meaning in the art, and relates to a base which, upon dissolution in water, does not fully dissociate.

The weak base may comprise any weak base wherein the solubility of said weak base is not impacted by temperature. Weak bases of this nature are advantageous as the neutralising agent will not easily fall out of solution if stored for long periods of time in cool storage conditions prior to use in methods as described herein.

The weak base may comprise a metal carbonate, a metal hydroxide, or a combination thereof. Examples of weak base include, but are not limited to, sodium carbonate, potassium carbonate, calcium carbonate, magnesium hydroxide, ferric hydroxide, or a combination thereof.

The neutralising agent may comprise sodium carbonate, potassium carbonate, calcium carbonate, or a combination thereof. The neutralising agent may comprise potassium carbonate. The reaction of an acid with a metal carbonate produces a salt, carbon dioxide, and water, meaning that a neutralising agent comprising a metal carbonate not only raises the pH of the treatment fluid described herein, such that it is suitable for immediate discharge directly to a municipal sewer, but also removes the biocidal effect of the treatment fluid described herein. Reducing the biocidal effect of the treatment fluid minimises the risk of corrosion of the sewer walls, and the production of unwanted gaseous by-products within the sewer.

When the neutralising agent comprises a strong base, the pH range of the neutralising agent may often be in the range of from about 12 to about 14. When the neutralising agent comprises a weak base, the pH range of the neutralising agent may often be in the range of from 9 to about 12.

The neutralising agent may be in the form of a solution or a suspension. As used herein, the term "solution" relates to liquid mixture in which the minor component(s) (I.e., the neutralising agent) is uniformly distributed throughout the major component (I.e., the diluent). The neutralising agent in the form of a solution may also be referred to as the neutralising solution. As used herein, the term "suspension" relates to a heterogeneous mixture of a liquid (i.e., the diluent) that contains solid particles (i.e., the neutralising agent) sufficiently large for sedimentation. The neutralising agent in the form of a suspension may also be referred to as a neutralising suspension. The diluent of the solution or suspension may comprise, consist essentially of or preferably consist of water.

The concentration of the neutralising solution or neutralising suspension may be in the range of from about 15% to about 30% by weight of the neutralising agent. The concentration of the neutralising solution or neutralising suspension may be in the range of from about 18% to about 23% by weight of the neutralising agent. It may be the case that the concentration of the neutralising solution or neutralising suspension is at least 20% by weight of the neutralising agent. It may be the case that the concentration of the neutralising solution or neutralising suspension is at least 21% by weight of the neutralising agent. It may be the case that the concentration of the neutralising solution or neutralising suspension is at least 22% by weight of the neutralising agent. It may be the case that the concentration of the neutralising solution or neutralising suspension is at least 23% by weight of the neutralising agent.

It may be the case that the neutralising solution or neutralising suspension is present in the range of from about 100 millilitres to about 600 millilitres. It may be the case that the neutralising solution or neutralising suspension is present in the range of from about 200 millilitres to about 500 millilitres.

The neutralising solution or neutralising suspension may be present in approximately the same volume as the disinfectant agent present in the disinfectant solution. For example, in instances where approximately 600 millilitres of disinfectant comprising peracetic acid and hydrogen peroxide was present in the disinfectant solution, approximately 600 millilitres of neutralising solution or neutralising suspension may be used. As used herein, the term "approximately" includes +/- 10% of the volume of disinfectant and/or neutralising solution/neutralising suspension.

The neutralising solution or neutralising suspension may be provided to the waste treatment system via a fluid delivery system, for example the third fluid delivery system 1530. The fluid delivery system 1530 may include a settling chamber 1534 similar to that described in relation to the first delivery system 1510. This may allow the neutralising solution or suspension to settle prior to introduction into the waste treatment system.

The neutralising solution or neutralising suspension may be provided via any of the first 1510 second 1520, and/or third 1530 fluid delivery systems, which may form a fluid delivery means 1500. It may be the case that at least some of the total volume of the diluent is provided by the second fluid delivery system 1520. Alternatively, it may be the case that the neutralising solution or neutralising suspension is provided in a 'pre-mixed' state via only one fluid delivery means into the waste processing vessel 120 (e.g., either the first fluid delivery system 1510, or the second 1520, or the third 1530, fluid delivery systems).

In one example, in a first step, the neutralising agent and a portion of the total volume of diluent may be provided to the waste processing vessel 120 via the first fluid delivery system 1510. A second component, such as the remaining volume of diluent, may be delivered to the waste processing vessel via the second fluid delivery system 1520 to achieve a desired mixture with desired relative concentrations of the neutralising solution or neutralising suspension. Alternatively, the neutralising agent and total volume of diluent may be added to the waste processing vessel in a single step via the first, the second fluid delivery system, or the third fluid delivery system.

In one example, the neutralising solution or neutralising suspension may be provided in a 'pre-mixed' state via a fluid delivery system, which may be any one of the first second or third fluid delivery system. Alternatively, in a first step, the neutralising agent and a portion of the total volume of diluent may be provided to the waste processing vessel 120 via the third fluid delivery system 1530. A second component, such as the remaining volume of diluent, may be delivered to the waste processing vessel via the second fluid delivery system 1520 to achieve a desired mixture with desired relative concentrations of the neutralising solution or neutralising suspension.

The neutralising agent may be in the form of a powder. As used herein, the term "powder" relates to a solid substance of fine, loose particles. The powder may be obtained using any known techniques, such as pulverisation, grinding, crushing, or disintegration. The particles may have a particle size in the range of from about 5 $\mu$m to about 500 $\mu$m. Particle size analysis of the powder can be determined using any known technique, such as dynamic image analysis (DIA), static laser light scattering, dynamic light scattering (DLS), sieve analysis, or by visual analysis of Transmission Electron Microscopy (TEM) or Scanning Electron Microscopy (SEM) images.

The neutralising agent may be in the form of a tablet. As used herein, the term "tablet" relates to a solid substance, which is often initially formed from a powder, that has been pressed or compacted into a solid dosage form. The tablet may have a wide range of geometries including, but not limited to, spherical, cubic, prismatic, pentagonal, hexagonal, heptagonal, or octagonal. The geometries will often be mathematically imperfect, such that they may be "substantially" of a given geometry. As used herein, regarding these geometries, the term "substantially" can be taken to mean clearly recognisable as a given geometry (for instance spherical), but not mathematically of that geometry (e.g., not a perfect sphere). This could include elongation along one axis by, perhaps, $\pm$20% or $\pm$10%, maybe in the range 20% or 10% to 1%.

When the neutralising agent is administered as a tablet or as a powder, it may be the case that, following completion of the waste shredding and liquid treatment process, the waste processing vessel 120 is orientated in the first position, in which the neutralising agent may be loaded into the waste processing vessel 120. This may include orienting the waste receiving opening 1270 of the waste processing vessel 120 towards a loading position and may include arranging the waste receiving opening 1270 towards the loading hatch 160 to enable a user to load the neutralising agent in the form of a tablet or a powder through the loading hatch 160 into the waste processing vessel 120 in the direction of arrow 1601. This can be seen in Figure 7A. The vessel may otherwise be opened for the neutralising agent to be added, which may happen while the vessel is in its second position.

Once the neutralisation agent has been added (either in the form of a tablet or powder, or in the form of a solution or suspension as described above), the rotatable blade arrangement may be rotated so as to carry out a mixing operation, to mix the shredded waste and the treatment fluid with the neutralising agent, and optional diluent, in the bottom of the waste processing vessel 120. Rotation of the blade arrangement allows for more efficient mixing of the neutralising agent throughout the waste and treatment fluid, resulting in a more efficient neutralisation step.

The blade arrangement may be rotated for a period of time in the range of from about thirty seconds to about 10 minutes. The blade arrangement may be rotated for a period of time in the range of from about 1 minute to about 8 minutes.

It may be the case that the neutralising agent is added to the treatment fluid once it has been emptied from the waste processing vessel.

It may be the case that the treatment fluid is separated from the shredded waste and emptied into a container. The container may be separate from or integral to the apparatus 1000 described herein. Alternatively, the container may be integrated into the apparatus described herein, such that it is fluidly connected to the waste processing vessel. The neutralising agent as described herein may be added to the treatment fluid in the container. In one example, wherein the neutralising agent is in the form of a solution or suspension, it may be the case that the first, second and/or third fluid delivery means described above are redirected to the container to allow for delivery of the neutralising solution or neutralising suspension. In another example, the neutralising solution or neutralising suspension may be prepared outside the waste processing apparatus described herein and added to the container. Alternatively, the treatment fluid may be emptied from the waste processing vessel directly into an exit conduit for delivery into an external sink, such as an external container, a drain or a municipal sewer. It may be the case that the neutralising agent is added to the exit conduit at the same time as the treatment fluid. Alternatively, the conduit may comprise an inlet for addition of the neutralising agent as described herein.

**Control System**

One aspect of improvement identified is controlling a shredding device to move into an operating position (which may be one of several operating positions) in which combined shredding and cleaning and/or disinfection of contaminated or biohazardous waste can take place simultaneously within the shredding device.

In one aspect, there is provided a control system for a waste processing apparatus. The control system comprises a controller configured to perform one or more of the following features: control an actuator of the waste processing apparatus to move a shredding device of the waste processing apparatus into at least a shred position in which a vessel of the shredding device is able to hold liquid; control a fluid delivery device of the waste processing apparatus to deliver a treatment fluid into the vessel of the shredding device; and control the shredding device to perform a shredding function to shred waste within the vessel of the shredding device.

The controller may be configured to receive sensor data from a sensor associated with the vessel, and determine, based on the sensor data, that the vessel is in the shred position.

The controller may be configured to, in response to determining that the vessel is in the shred position, control a closure mechanism of the waste processing apparatus to move a closure arrangement to close the vessel of the shredding device.

The controller may be configured to, in response to determining that the vessel is in the shred position, control one or more locking mechanisms of the waste processing apparatus to lock the shredding device in the shred position.

The controller may be configured to control the actuator to move the shredding device into the shred position in response to receiving an input signal from a user input device of the waste processing apparatus.

The controller may be configured to control the fluid delivery device to deliver a predefined amount of treatment fluid into the vessel of the shredding device.

The waste processing apparatus may comprise a blade arrangement motor configured to drive a blade arrangement of the shredding device to perform the shredding function. The controller may be configured to control the blade arrangement motor to control the rotation speed and/or rotation direction of the blade arrangement.

The controller may be configured to control the blade arrangement motor to alternate between different rotation directions.

The controller may be configured to: control the blade arrangement motor to drive the blade arrangement in a first mode in which the blade arrangement rotates at a first speed, and in response to determining that the torque of the blade arrangement motor has decreased below a first threshold torque value, control the blade arrangement motor to drive the blade arrangement in a second mode in which the blade arrangement motor rotates at a second speed greater than the first speed.

The controller may be configured to control the shredding device to terminate the shredding function after a predefined shredding time has elapsed and/or in response to determining that a torque value of the shredding device is below a second threshold torque value.

The controller may be configured to: control the actuator to move the shredding device into an emptying position in which waste can be emptied out of the vessel of the shredding device, and control a conveying means to move said waste into a waste bin.

The controller may be configured to control a closure mechanism of the waste processing apparatus to move a closure arrangement to close the vessel of the shredding device.

The controller may be configured to control one or more locking mechanisms of the waste processing apparatus to lock the shredding device in the shred position.

The controller may be configured to initiate a neutralisation process. The neutralisation process may include activating the waste processing apparatus to deliver a neutralisation agent to the treatment fluid. The neutralisation process may include opening the vessel to allow a neutralisation agent to be added into the vessel. The neutralisation process may include controlling a fluid delivery device so as to deliver a neutralising agent into the vessel. The controller may be configured to control the fluid delivery device to deliver the neutralising agent into the vessel after terminating the shredding function of the shredding device. The neutralisation process may include delivering a neutralising agent to the treatment fluid outside of the vessel.

The controller may be configured to permit the waste processing apparatus to perform a waste processing operation in response to verifying one or more of the following conditions: a user has entered valid credentials for operating the waste processing apparatus; a water pressure of a water supply for the waste processing apparatus is at least a minimum pressure value; and a level of treatment fluid in a treatment fluid container of the waste processing apparatus is sufficient.

The controller may be configured to control the shredding device to perform the shredding function in response to verifying one or more of the following conditions: a loading hatch of the waste processing apparatus is closed; the shredding device is in the shred position; the vessel of the shredding device is closed by a closure arrangement; the shredding device is locked in the shred position by one or more locking mechanisms; a predefined amount of treatment fluid has been added to the vessel; and a predefined amount of water has been added to the vessel.

The controller may comprise one or more processors.

In another aspect, there is provided a waste processing apparatus comprising one or more of the following features: a shredding device comprising a vessel for holding waste and treatment fluid during processing, wherein the shredding device is configured to perform a shredding function to shred waste within the vessel; an actuator configured to move the shredding device into a shred position in which the vessel is able to hold liquid; a fluid delivery device configured to deliver a treatment fluid into the vessel of the shredding device; and a control system as set out above. The controller of the control system is communicatively coupled to the shredding device, the actuator and the fluid delivery device.

The actuator may comprise a motor configured to rotate the shredding device into at least the shred position.

The fluid delivery device may comprise: a container for holding treatment fluid; and a pump configured to pump treatment fluid from the container into the vessel of the shredding device.

In another aspect, there is provided a method of controlling a waste processing apparatus. The method comprises one or more of the following features: controlling an actuator of the waste processing apparatus to move a shredding device of the waste processing apparatus into a position in which the shredding device is able to hold liquid; controlling a fluid delivery device of the waste processing apparatus to deliver a treatment fluid into the shredding device;

and controlling the shredding device to perform a shredding function to shred waste within the shredding device.

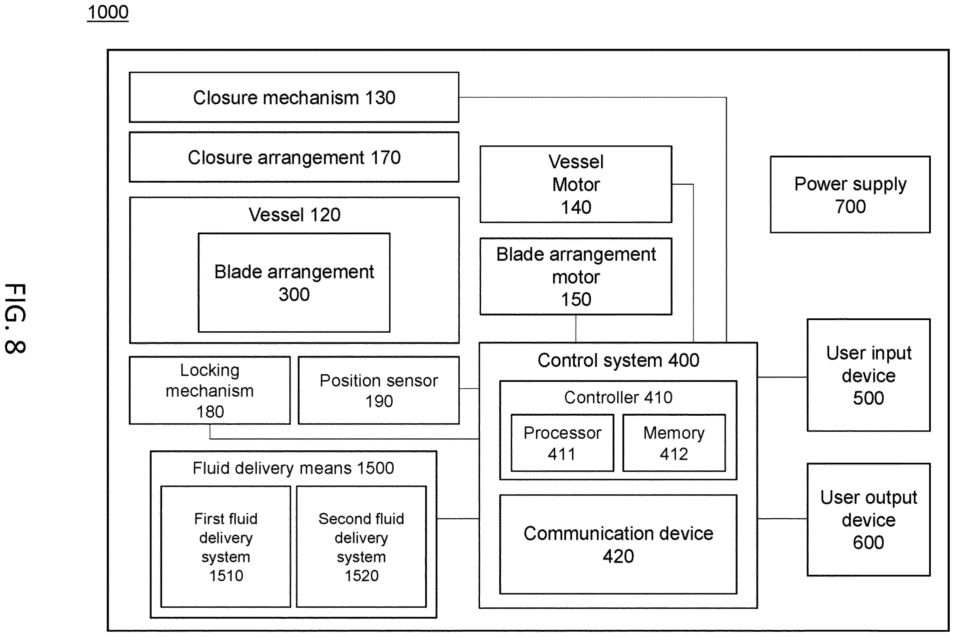

In another aspect, there is provided a computer-readable medium comprising instructions which, when executed by a processor, cause the processor to perform one or more of the following features: control an actuator of a waste processing apparatus to move a shredding device of the waste processing apparatus into a position in which the shredding device is able to hold liquid; control a fluid delivery device of the waste processing apparatus to deliver a treatment fluid into the shredding device; and control the shredding device to perform a shredding function to shred waste within the shredding device. Figure 8 is a block diagram showing various components of the waste processing apparatus 1000 shown in Figure 1. The waste processing apparatus 1000 includes a control system 400 which controls the overall operation of the waste processing apparatus 1000. One or more components of the control system 400 may be disposed within the control system enclosure 1004 of the waste processing apparatus 1000. Figure 16 shows various connections between the control system 400 and other components of the waste processing apparatus 1000. However, these connections are not intended to be limiting, and other connections could be used. The control system 400 may be connected to one or more components directly (i.e. with no intervening components), or may be connected to one or more components via other components of the waste processing apparatus 1000.

The control system 400 includes a controller 410. The controller 410 may be configured to control the waste processing apparatus 1000 to perform any of the functions or processes described herein. The controller 410 is configured to control the vessel motor 140 to move the vessel 120 into various positions, such as the positions described above with reference to Figures 6A to 6C. In particular, the controller 410 is configured to control the vessel motor 140 to move the vessel into a shredding position, which may be the same as, or similar to, the second position described above in relation to Figure 6B. The controller 410 may be the same as the controller 410 described in relation to the fluid delivery system 1510 shown in Figures 5A and 6A. It will be appreciated that any or all features described in relation to the controller 410 may apply to the controller 410 described in relation to the fluid delivery system 1510. In alternative arrangements, the controller 410 of the fluid delivery system 1510 may be separate to the controller 410 of the control system 400, but may still comprise any or all of the features of the controller 410 of the control system 400. The controller 410 is configured to, when the vessel 120 is in the shredding position, control the fluid delivery apparatus 1500 to deliver treatment fluid into the waste processing vessel 120, and control the shredding device to perform a shredding function to shred waste within the vessel 120. This allows for simultaneous treatment and shredding of the waste.

In some examples, the controller 410 may include a memory 412 and one or more processors 411 configured to execute instructions stored in the memory to perform control operations, such as moving the vessel 120 into a given position. In other examples, the controller 410 may be implemented using hardware. In some cases, the controller 410 may perform control operations based on a user input. Such a user input may be received from an input device of the waste processing apparatus 1000. Alternatively, or in addition, the controller 410 may perform control operations based on input received from an external system.

In the present example, the controller 410 is a programmable logic controller (PLC). The PLC 410 includes a processor 411 and a memory 412. The processor 411 is configured to receive signals from input devices (such as sensors) and make decisions based on a program stored in the memory 412 to control devices such as the vessel motor 140. The memory 412 may store instructions that are executable by the processor 411 to perform any of the operations described herein.

The control system 400 includes a communication device 420 which enables communication between the controller 410 and various devices in the waste processing apparatus 1000, such as the vessel motor 140. In the present example, the communication device 420 is an ethernet switch. The communication device 420 may also allow for communication between the controller 410 and one or more external devices or systems. For example, the communication device 420 may be connected to a cloud data storage system via a router (not shown), and the controller 410 can send data to the cloud data storage system via the router. In some cases, the router may buffer the data locally and periodically send the data to the cloud data storage system. If an internet connection is not present, data may be buffered locally in the memory 412 until the communication device 420 can connect to the cloud data storage system again. The data can then uploaded to the cloud data storage system from the memory 412. Data stored in the cloud data storage system may be used for (for example) online dashboards, automatic reporting or automatic service ticketing.

The waste processing apparatus 1000 includes at least one user input device 500 and at least one user output device 600. The input device 500 allows the user to control various operations of the waste processing apparatus 1000. The input device may be, for example, a button (e.g. an emergency stop button) or a switch. The output device 600 provides the user with information about the state of the waste processing apparatus, e.g. the current processing cycle that is in progress. The output device 600 may be, for example, a display device. In some examples, the input device 500 and the output device 600 may be implemented together in the same device, such as a touch screen display device.

The waste processing apparatus 1000 includes a power supply 700 which is configured to supply direct current (DC) power to electrical components of the waste processing apparatus 1000, such as the vessel motor 140 and the blade arrangement motor 150.

The controller 410 may be configured to determine the current position of the vessel 120 based on data from a sensor 190 which is associated with the vessel 120. The sensor 190 may be a position sensor, or may be an image sensor such as a camera. In the present example, the sensor 190 is a rotary encoder which outputs a signal indicating the absolute angular position of the shaft of the vessel motor 140. In examples where an image sensor is used, the controller 410 may analyse images received from the image sensor to determine the position of the vessel 120.

The controller 410 can determine that the vessel 120 is in a given position (e.g. the shred position) based on the data from the sensor 190. For example, if the position signal from the rotary encoder indicates that the angular position of the shaft is 5°, then the controller 410 may determine that the vessel 120 is in the shred position. Data specifying the correspondence between different angular positions and different operational positions of the vessel 120 may be stored in the memory 412.

The controller 410 may control the closure mechanism 130 and/or the locking mechanisms 180 based on feedback from the sensor 190. In the present example, the controller 410 is configured to control the closure mechanism 130 to move the closure arrangement 170 into the closed position only when it has determined that the vessel 120 is in the shred position. Similarly, the controller 410 is configured to control the locking mechanisms 180 to lock the vessel 120 in position only when it has determined that the vessel 120 is in the shred position. This reduces the chance of errors occurring during the engagement of the closure arrangement 170 and/or the locking mechanisms 180. In other examples, the controller 410 may control the closure arrangement 170 and/or the locking mechanisms 180 without relying on feedback from the sensor 190, or the sensor 190 may be omitted altogether.

The controller 410 is configured to control the fluid delivery means 1500 to deliver one or more fluids into the vessel 120. The controller 410 may be configured to control the fluid delivery apparatus 1500 to deliver predefined amounts of the one or more fluids into the vessel 120. The amount(s) of fluid(s) delivered into the vessel 120 may be determined by the controller 410 based on feedback from a flowmeter, e.g. flowmeter 1515 and/or based on signals from the sensor arrangement 1546 as discussed above.

In the present example, controller 410 is configured to control the first fluid delivery system 1510 of the fluid delivery means 1500 to deliver treatment fluid into the chamber 1201 of the vessel 120 via the settling vessel 1534. In the present example, the controller 410 is configured to control the first fluid delivery system 1510 of the fluid delivery means 1500 to deliver treatment fluid into the chamber 1201 of the vessel 120 only when it has determined that the vessel 120 is locked in position and the closure arrangement 170 is covering the vessel 120. In the present example, the controller 410 is configured to control the first and second fluid delivery means 1536, 1538 so as to deliver a predefined amount of the treatment fluid into the chamber 1201, based on feedback from the sensor arrangement 1546 (and the flowmeter 1515 in some examples). The predefined amount of treatment fluid may be any of the amounts set out herein.

In the present example, the controller 410 is also configured to control the second fluid delivery system 1520 so as to deliver a predefined amount of diluent (which may be water) into the chamber 1201, based on feedback from the flowmeter 1515 and/or a sensor arrangement coupled to a settling vessel of the second fluid delivery system 1520. The predefined amount of diluent may be any of the amounts set out herein. This results in the liquid mixture in the chamber 1201 having a particular desired concentration of treatment fluid, which may be any of the concentrations set out herein. Data defining the predefined amounts of treatment fluid and/or diluent may be stored in the memory 412 of the controller 410.

In some examples, the controller 410 may be configured to initiate a neutralisation process. The neutralisation process may include activating the waste processing apparatus 1000 to deliver a neutralisation agent to the treatment fluid. The neutralisation process may include controlling the closure mechanism 130 to open the vessel 120 to allow a neutralisation agent to be added into the chamber 1201, or alternatively may include controlling any one of the first to third fluid delivery systems 1510, 1520, 1530 so as to deliver a neutralising agent into the chamber 1201. The controller 410 may be configured to control one of the first to third fluid delivery system 1510, 1520, 1530 to deliver the neutralising agent into the chamber 1201 after terminating the shredding function of the shredding device. Alternatively, or in addition, the neutralisation process may include delivering neutralising agent to the treatment fluid outside of the vessel 120. For example, the treatment fluid may be separated from the shredded waste and collected in a container or conduit. An example of such a conduit may be a pipe, tube or channel for directing fluid away from the waste processing apparatus 1000 towards, for example, a drain or other external sink. The neutralisation process may include adding the neutralisation agent to the treatment fluid in the container or conduit after it has been separated from the shredded waste. A predefined amount of neutralising agent may be used for the neutralisation process. The predefined amount may be measured based on feedback from a flowmeter. The predefined amount of neutralising agent may be any of the amounts set out herein. Data defining the predefined amount of neutralising agent may be stored in the memory 412 of the controller 410.

The controller 410 is configured to control the blade arrangement motor 150 of the shredding device to rotate the blade arrangement 300 within the vessel 120, in particular when the treatment fluid is present in the chamber 1201. In the present example, the controller 410 is configured to control the blade arrangement motor 150 to rotate the blade arrangement 300 within the vessel 120 only when it has determined that the correct amount of treatment fluid (and in the present case, the correct amount of water) has been added to the chamber 1201.

During operation of the blade arrangement motor 150, the blade arrangement motor 150 will have a given torque, related to the resistance to the blade motion of the waste it is shredding, and the controller 410 may be able to determine a value of the torque by various means. For example, the controller 410 may estimate a torque value of the blade arrangement motor 150 based on the current being used by the blade arrangement motor 150, or may receive a torque value of the blade arrangement motor 150 from a torque sensor (not shown).

The controller 410 can control the blade arrangement motor 150 so as to control, for example, the rotation speed and/or rotation direction of the blade arrangement 300. The controller 410 may select the rotation speed of the blade arrangement 300 based on the torque of the blade arrangement motor 150. For example, the torque of the blade arrangement motor 150 may be relatively high at the beginning of a waste processing cycle due to the high force required to shred the waste. At this stage, the controller 410 may control the blade arrangement motor 150 to rotate the blade arrangement 300 at a low speed, e.g. 10-20 rotations per minute (RPM). As the waste is shredded, the force required to shred the waste decreases, and so the torque of the blade arrangement motor 150 decreases. In response to detecting that the torque of the blade arrangement motor has decreased below a threshold torque value, the controller 410 may increase the rotation speed of the blade arrangement motor 150, e.g. to 30-40 RPM. For example, if the controller 410 estimates the torque of the blade arrangement motor 150 based on the current being used by the blade arrangement motor 150, the controller may increase the rotation speed of the blade arrangement motor 150 in response to the current per phase falling below 10 Amps per phase. At low rotation speeds, smaller pieces of waste may escape the cutting action of the blades, whereas this is less likely at higher speeds. Driving the blade arrangement 300 at an increased speed thus helps to ensure that the waste is shredded to a small particle size. Driving the blade arrangement 300 at an increased speed also helps to effectively mix the waste and the treatment fluid.

In some examples, the controller 410 may control the blade arrangement motor 150 to alternate between rotation in different directions, so as to rotate the blade arrangement 300 in different rotational directions. This allows the blade arrangement 300 to shred waste in both rotational directions. This reduces the force required to achieve complete shredding of the waste compared to shredding in a single rotational direction only, thereby reducing the power required to drive the blade arrangement 300. The rotation direction of the blade arrangement motor 150 may be adjusted in combination with adjusting the rotation speed. The controller 410 may control the blade arrangement motor 150 to periodically alternate between rotation in different directions. Reversing the direction of the blade arrangement 300 periodically has been found to reduce the incidence of waste collecting in a single area of the vessel 120, and may facilitate more efficient shredding. Alternatively, or in addition, the controller 410 may control the blade arrangement motor 150 to change rotation direction in response to determining that the torque value of the blade arrangement motor 150 has exceeded a maximum value (e.g. the current being used by the blade arrangement motor 150 has exceeded a maximum current value, or the torque measured by a torque sensor has exceeded a maximum value). This may, for instance, indicate that the blade arrangement 300 has encountered a hard object, e.g. a piece of steel, which it cannot immediately shred. Reversing the rotation direction may allow for the object to enter the blade arrangement 300 in a different orientation which allows for easier shredding.

In some examples, the controller 410 is configured to control the blade arrangement motor 150 to rotate the blade arrangement 300 for a predefined shredding or cycle time, i.e. the blade arrangement 300 stops rotating after the predefined shredding time has elapsed. Data defining this predefined shredding time may be stored in the memory 412 of the controller 410. Alternatively, or in addition, the controller 410 may be configured to control the blade arrangement motor 150 to rotate the blade arrangement until the controller 410 determines that the torque of the blade arrangement motor 150 has fallen below a minimum torque value (e.g. the current being used by the blade arrangement motor 150 has fallen below a minimum current value, such as 6 Amps per phase, or the torque measured by a torque sensor has fallen below a minimum value). Data defining this minimum torque value may be stored in the memory 412 of the controller 410.

In examples where a neutralising agent is added to the vessel 120, the controller 410 may be configured to control the blade arrangement motor 150 to rotate the blade arrangement 300 for a predefined period of time to mix the shredded waste and the treatment fluid with the neutralising agent, and optional diluent, in the bottom of the waste processing vessel 120. The controller 410 may control the blade arrangement motor 150 to rotate the blade arrangement 300 for a period of time in the range of from about thirty seconds to about 10 minutes, or from about 1 minute to about 8 minutes.

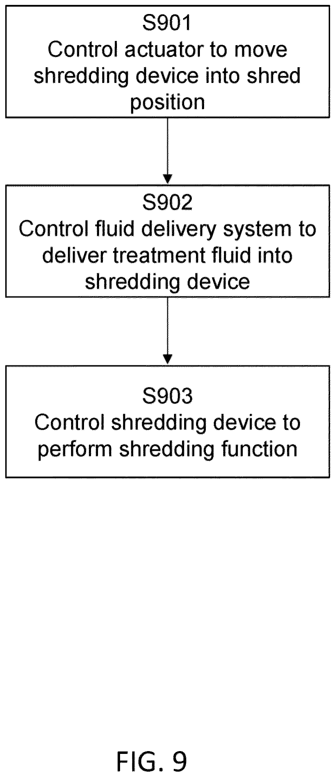

An example of a method of controlling the waste processing apparatus 1000 is shown in Figure 9. The method includes controlling an actuator of the waste processing apparatus to move a shredding device of the waste processing apparatus into a position in which the shredding device is able to hold liquid (S901). The method further includes controlling a fluid delivery system 1510 of the waste processing apparatus 1000 to deliver a treatment fluid into the shredding device (S902). The method further includes controlling the shredding device to perform a shredding function to shred waste within the shredding device (S903).

Example operating cycles of the waste processing apparatus 1000 will now be described.

In some circumstances, the controller 410 may verify that certain operating conditions are met before commencing these cycles. If at least one of these operations conditions is not met, the controller 410 will not begin processing. For example, the controller 410 may verify that the water pressure is at least a minimum pressure value (in this case, 1 bar), based on feedback from a water pressure sensor (not shown). Alternatively, or in addition, the controller 410 may verify that the level of treatment fluid in the container of the fluid delivery apparatus is at least a minimum or predetermined volume (in this case, 0.1 litres), based on feedback from a level sensor (not shown). In some examples, the user may need to present valid security credentials (e.g. via the input device 500) to operate the waste processing apparatus 1000, and the controller 410 will not begin processing until such credentials are received.

**Fluid Delivery Cycle**

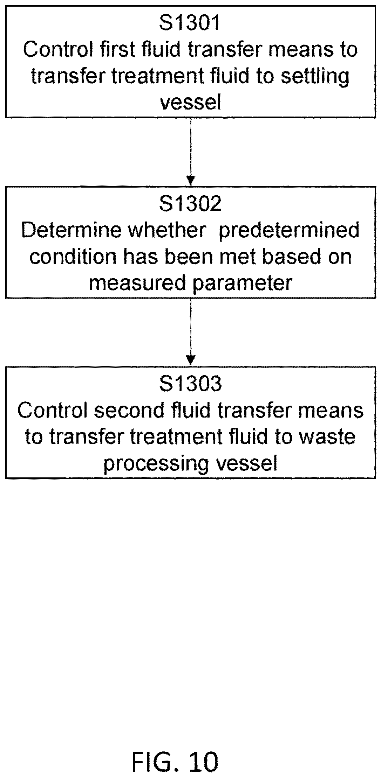

An example of a method of controlling the fluid delivery system 1510 (e.g. step S902 of Figure 9) is shown in Figure 10. In the present example, the controller 410 is configured to control the first fluid transfer means 1536 to transfer a treatment fluid to the settling vessel 1534 configured to retain the fluid (S1301). The treatment fluid may be transferred from the fluid reservoir 1511. Controlling the first fluid transfer means 1536 may include controlling the pump 1513 and/or the valve 1514. This step may be performed by the controller 410 generating the third output as discussed above. The third output may include instructions from the controller 410 to the first fluid transfer means 1536 to deliver a predetermined volume of treatment fluid to the settling vessel 1534. The controller 419 may determine whether the volume in the settling vessel 1534 is greater than or equal to the predetermined volume based on a signal from the sensor arrangement 1546. If the volume is less than the predetermined volume, the controller 410 may generate a fourth output to indicate to an operator that the reservoir 1511 is empty and/or that there is a fault in the first fluid transfer means 1536.

The controller 410 may next determine whether a predetermined condition associated with the fluid in the settling vessel 1534 has been met based on the measured parameter associated with the fluid in the settling vessel 1534 (S1302). This step may include the controller 410 obtaining a signal from the sensor arrangement 1546 that may indicate the level or volume of fluid in the settling vessel 1534. This step may be performed in between waste processing cycles of the waste processing system so as to facilitate more accurate readings from the sensor arrangement 1546. The step may include the controller 410 determining an elapsed time from the introduction of the fluid to the settling vessel 1534 and comparing the elapsed time with a minimum settling time (e.g. stored in the memory 412).

The controller 410 may compare the volume of fluid in the settling vessel 1534 with the predetermined volume based on the signal from the sensor arrangement 1546. The controller 410 may do this after determining that the minimum settling time has elapsed. If the volume of fluid is less than the predetermined volume (e.g. as a result of gas bubble dissipation causing the level or volume to decrease), the controller 410 may instruct the first fluid transfer means 1536 to deliver additional fluid into the settling vessel 1534 until the volume is greater than or equal to the predetermined volume. The controller 410 may generate the third output to instruct the first fluid transfer means 1536 to introduce additional fluid. If the volume is still less than the predetermined volume at this stage (e.g. after generating the third output), the controller may generate the fourth output to indicate to an operator that the reservoir 1511 is empty and/or there is a fault in the first fluid transfer means 1536.

The controller 410 may detect, or receive an input indicating, the status of the waste treatment cycle in the vessel 120. In some arrangements, once the waste treatment cycle is completed, and the volume of treatment fluid in the settling vessel 1534 is at or above the predetermined volume, and the minimum settling time has elapsed, the controller 410 may determine that the predetermined condition has been met. It will be appreciated that, in some arrangements, any combination of the above conditions in any order may be required to meet the predetermined condition. In an example arrangement, the controller 410 may first determine that the minimum settling time has elapsed, before then determining that the predetermined volume of treatment fluid is present, before then determining that the waste treatment cycle of the waste treatment system is completed. In some arrangements, alternative or additional predetermined conditions may be required to be met, including rate of change of volume, or level or rate of change of a level of an upper surface, of the treatment fluid, temperature, pressure of the treatment fluid or any other properties associated with the treatment fluid in the settling vessel 1534.

Once the predetermined condition has been met, the controller 410 may control a second fluid transfer means 1538 to transfer the treatment fluid from the settling vessel 1534 to the waste processing vessel 120 (S1303). Controlling the second fluid transfer means 1538 may include controlling a pump and/or the valve 1540 in the fluid conduit 1542. This step may be performed by the controller 410 generating the first output. This ends the fluid delivery cycle.

The controller 410 may be configured to determine that all of the treatment fluid or a desired volume of treatment fluid has been transferred from the settling vessel 1534 to the waste processing vessel 120 (e.g. by detecting that the settling vessel 1534 is empty) and in response thereto generate the third output to deliver fresh treatment fluid from the reservoir 1511 to the settling vessel 1534 to recommence the fluid delivery cycle. The third output may be generated manually by an operator. Steps S1301 to S1303 may occur while the waste cycle is not in operation (e.g. between cycles, when the blade arrangement is not operating) to facilitate more effective measurements.

A computer-readable medium may comprise instructions which, when executed by a processor, cause the processor to perform steps S1301, S1302 and S1303. It will be appreciated that the processor may be configured to perform any combination of the above additional steps of the fluid delivery cycle.

It will be understood that although the fluid delivery cycle and computer-readable medium have been described as delivering a treatment fluid to the waste delivery apparatus 1000 described herein, the fluid delivery cycle and computer-readable medium may be utilised in delivering a treatment fluid to any waste treatment system, for example any biohazardous waste treatment system.

**Waste Cycle**

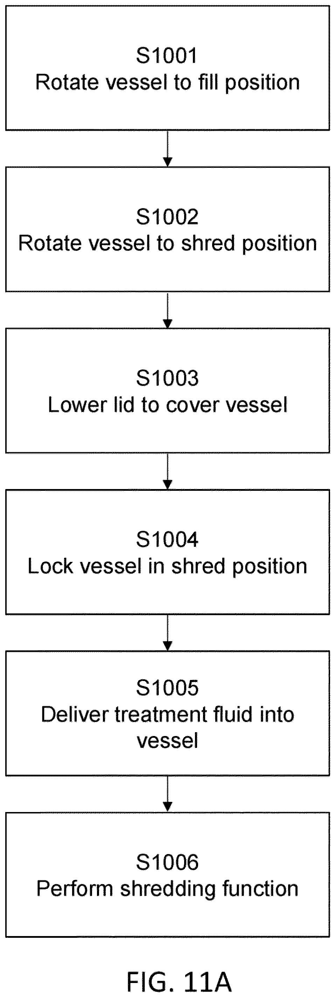

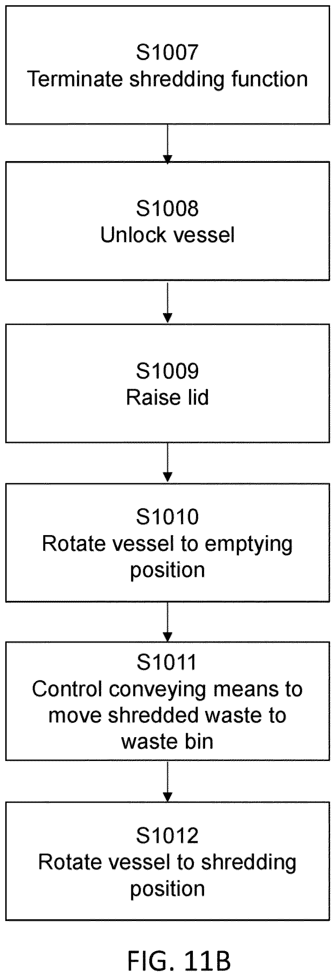

An example of a waste cycle process is shown in Figures 11A and 11B. In the present example, in response to receiving a selection of an "Enable fill position" button from a touch screen display, the controller 410 controls the vessel motor 140 to rotate the vessel 120 to the fill position (S1001) in which the waste receiving opening 1270 of the vessel 120 is presented to the loading hatch 160. In the present case, the rotational position of the vessel 120 in the fill position is 50° from vertical. The controller 410 may determine that the vessel 120 is in the fill position based on feedback from the vessel position sensor 190. In the present example, the controller 410 controls the touch screen display to make a button "Open door" appear grey, and in response to receiving a selection of the button "Open door" from the touch screen display, unlocks the loading hatch 160. The operator opens the loading hatch 160 and loads the vessel 120 with waste to be treated, then closes the loading hatch 160.

The controller 410 determines that the loading hatch 160 has been closed, and controls the touch screen display to change the colour of a button "Lock door and run" to grey. In the present example, in response to receiving a selection of the button "Lock door and run" from the touch screen display, the controller 410 locks the loading hatch 160 and controls the vessel motor 140 to rotate the vessel to the shred position (S1002). The rotational position of the vessel in the fill position may be vertical (i.e. 0 degrees), or close to vertical. In the present case, the rotational position of the vessel 120 in the shred position is 5°.

The controller 410 verifies that the vessel 120 is in the shred position based on feedback from the vessel position sensor 190. In response to verifying that the vessel 120 is in the shred position, the controller 410 controls the lid closure mechanism 130 to lower the lid 170 to cover the vessel 120 (S1003). The controller 410 also controls the linear actuators of the locking mechanisms 180 to move the locking pins 182 into their locking positions, so as to lock the vessel 120 to the chassis 1001 (S1004). In the present example, steps S403 and S404 are performed simultaneously. In other examples, these steps may be performed sequentially.

The controller 410 receives position signals from the lid actuator 130 and the locking mechanisms 180, and determines whether the lid 170 and the locking pins 182 are in the correct positions based on these signals. Once the controller 410 has verified that both the lid 170 and the locking pins 182 are in their positions, the controller 410 controls the fluid delivery means 1500 (e.g. fluid delivery system 1510) to deliver treatment fluid (and in the present case, water) into the vessel (S1005). The controller 410 may verify that the correct amounts of treatment fluid and water have been delivered into the vessel 120, based on feedback from flow meters.

The controller 410 controls the blade arrangement motor 150 to rotate the blade arrangement 300 to perform the shredding function (S1006). In the present example, the controller 410 controls the blade arrangement motor 150 to rotate the blade arrangement 300 of the shredding device to perform the shredding function for a predefined shredding time (in the present case, 12 minutes). The controller 410 may control the blade arrangement motor 150 to alternately rotate in forward and reverse directions, thereby alternating the rotating direction of the shredding blades.

Once the controller 410 has determined that the shredding time has elapsed, and that the blade arrangement 300 meets little or no resistance (based on the torque of the blade arrangement motor 150), the controller 410 stops the blade arrangement motor 150, thereby terminating the shredding function (S1007). In the present example, the resistance encountered by the blade arrangement 300 is inferred from the current being consumed by the blade arrangement motor 150. As an example, the current of the blade arrangement motor 150 under load conditions may vary between a first current level, such as 6 Amps per phase and a second current level, such as 13 Amps per phase. In this case, if the shredding function has been performed for the shredding time period, such as 12 minutes and the current being consumed by the blade arrangement motor 150 is less than a first current level, such as 6 Amps per phase, then the controller 410 may stop the blade arrangement motor 150.

The controller 410 then controls the linear actuators of the locking pin mechanisms 180 to move to the unlock positions to unlock the vessel (S1008), and controls the lid actuator 130 to raise the vessel lid 170 (S1009).

The controller 410 then controls the vessel motor 140 to rotate the vessel 120 into the emptying position in which waste can be evacuated from the vessel 120 (S1010). This may also be referred to as the fluid dispensing step, in which treatment fluid is removed from the vessel 120. In the present case, the rotational position of the vessel 120 in the emptying position is 180° with respect to vertical. The controller 410 can verify that the vessel 120 is in the emptying position based on feedback from the vessel position sensor 190, in the same way as for the fill position and the shred position.

Since the opening 1270 of the vessel 120 is uncovered in the emptying position, the shredded waste falls out of the vessel 120 onto a conveying means (e.g. an auger). The controller 120 may also control the blade arrangement motor 150 to rotate the blade arrangement 300 to help move the shredded waste out of the vessel 120. The controller 410 controls the conveying means to transport the shredded waste to a waste bin (S1011). Once a predefined time has elapsed, the controller 410 controls the vessel motor 140 to rotate the vessel 120 to the shredding position (S1012).

In some examples, a wash cycle is carried out once the vessel 120 has returned to the shredding position. An example of a wash cycle will now be described. With the vessel 120 in the shredding position, the controller 410 controls the locking mechanisms 180 to engage the locking pins 182 and controls the lid closure mechanism 130 to lower the lid 170 to cover the vessel 120. The controller 410 then controls the second fluid delivery means 1520 to deliver water into the vessel 120. The controller 410 also controls the blade arrangement motor 150 to rotate the blade arrangement 300 for a predefined cleaning time (in the present case, 60 seconds). This helps to clean the vessel 120. After the predefined time has elapsed, the controller 410 controls the locking mechanisms 180 to retract the locking pins 182 and controls the lid closure mechanism 130 to raise the lid 170. The controller 410 controls the vessel motor 140 to rotate the vessel 120 to the emptying position, where the water will be drained out of the vessel 120. The controller 410 controls the vessel motor 140 to return the vessel 120 to the shred position, controls the locking pin mechanisms 180 to engage the locking pins 182 and controls the lid 170 to cover the vessel 120. The ends the wash cycle.

**Validation Cycle**

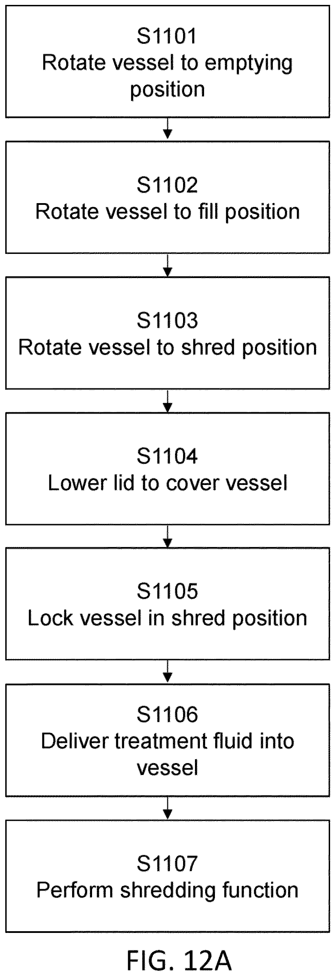

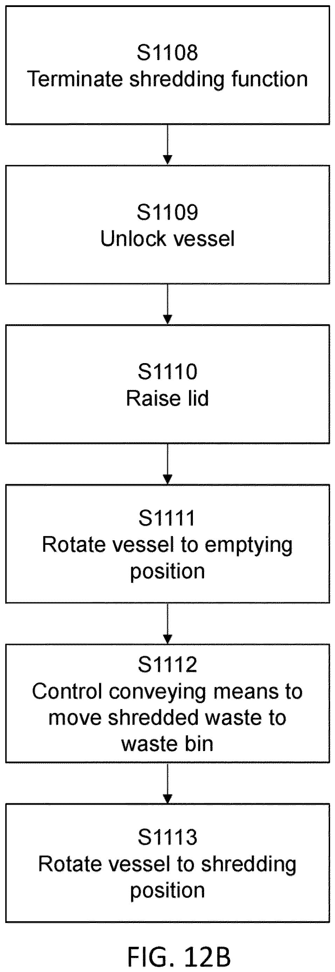

An example of a validation cycle process is shown in Figures 12A and 12B. In the present example, in response to receiving a selection of an "Enable Validation" button from the touch screen display, the controller 410 controls the vessel motor 140 to rotate the vessel 120 to the emptying position (S1101). In this present case, this emptying position is the same as the emptying position described above in relation to the waste cycle. The controller 410 can verify that the vessel 120 is in the emptying position based on feedback from the vessel position sensor 190. In the emptying position, the validation ports 1261, 1262, 1263 on the underside of the vessel 120 face the loading hatch 160 of the waste processing apparatus. In the present example, the controller 410 controls the touch screen display to make the button "Install manifold" appear grey, and in response to receiving a selection of the button "Install manifold" from the touch screen, unlocks the loading hatch 160. The operator opens the loading hatch 160 and loads validation plugs, with biological indicator strips installed, into the validation ports 1261, 1262, 1263. The operator then closes the loading hatch 160.

The controller 410 can determine that the loading hatch 160 has been closed and controls the touch screen display to change the button "Manifold fitted" to appear grey. In response to receiving a selection of the button "Manifold fitted" from the touch screen display, the controller 410 locks the loading hatch 160. The controller 410 controls the vessel motor 140 to rotate the vessel 120 to the fill position (S1102). The controller 410 can determine that the vessel is in the fill position based on feedback from the vessel position sensor 190. In the present example, the controller 410 controls the touch screen display to make the button "Open door" appear grey, and in response to the receiving a selection of the button "Open door" from the touch screen display, unlocks the loading hatch 160. The operator opens the loading hatch 160 and loads the vessel with the waste to be treated, then closes the loading hatch 160.

The controller 410 determines that the loading hatch 160 has been closed, and controls the touch screen display to change the colour of the button "Lock door and run" to grey. In response to receiving a selection of the button "Lock door and run" from the touch screen display, the controller 410 locks the loading hatch 160. The controller 410 controls the vessel motor 140 to rotate the vessel 120 to the shred position (S1103).

The controller 410 verifies that the vessel 120 is in the shred position based on feedback from the vessel position sensor 190. Once the controller 410 has verified that the vessel 120 is in the shred position, the controller 410 controls the lid closure mechanism 130 to lower the lid 170 to cover the vessel (S1104). The controller 410 also controls the linear actuators of the locking pin mechanisms 180 to move the locking pins 182 to lock the vessel 120 to the chassis 1001 (S1105). In the present example, these steps are performed simultaneously. In other examples, these steps may be performed sequentially.

The controller 410 receives position signals from the lid actuator and the locking pin mechanism 180, and determines whether the lid 170 and the locking pins 182 are in the correct positions based on these signals. Once the controller 410 has verified that both the lid 170 and the locking pins 182 are in their positions, the controller 410 controls the fluid delivery means 1500 to deliver treatment fluid (and in the present case, water) into the vessel (S1106). The controller 410 may verify that the correct amounts of treatment fluid and water have been delivered into the vessel 120, based on feedback from flow meters and signal arrangements.

The controller 410 controls the blade arrangement motor 150 to rotate the blade arrangement 300 to perform the shredding function (S1107). In the present example, the controller 410 controls the blade arrangement motor 150 to rotate the blade arrangement 300 to perform the shredding function for a predefined shredding time (in the present case, 12 minutes). As with the waste cycle, the controller 410 may control the blade arrangement motor 150 to alternately rotate in forward and reverse directions, thereby alternating the rotating direction of the shredding blades.

Once the controller 410 has determined that the predefined shredding time has elapsed, and that the blade arrangement 300 meets little or no resistance (based on the torque of the blade arrangement motor 150), the controller 410 stops the blade arrangement motor 150 to terminate the shredding function (S1108). In the present example, the resistance encountered by the blade arrangement 300 is inferred from the current being consumed by the blade arrangement motor 150. As an example, the current of the blade arrangement motor 150 under load conditions may vary between a first current level, such as 6 Amps and a second current level, such as 13 Amps per phase. In this case, if the shredding function has been performed for the shredding time period, such as 12 minutes, and the resistance, which may be indicated by a current being consumed by the blade arrangement motor 150, is less than a first current level, such as 6 Amps per phase, then the controller 410 may stop the blade arrangement motor 150.

The controller 410 then controls the linear actuators of the locking pin devices to move to the unlock positions to unlock the vessel 120 (S1109), and controls the lid closure mechanism 130 to raise the vessel lid 170 (S1110).

The controller 410 then controls the vessel motor 140 to rotate the vessel 120 into the emptying position (S1111). The controller 410 can verify that the vessel 120 is in the emptying position based on feedback from the vessel position sensor 190, in the same way as for the fill position and the shred position. The shredded waste falls from the vessel onto the auger. The controller 410 controls the conveying means to transport the shredded waste to the waste bin (S1112).

With the vessel 120 in the emptying position, the controller 410 controls the touch screen display to change the button "Test strip removed" to grey. The controller 410 then unlocks the loading hatch 160. The operator opens the loading hatch 160 and removes the validation plugs, and replaces the validation plugs with blanking plates. In response to receiving a selection of the "Test strip removed button" from the touch screen display, the controller 410 locks the loading hatch 160. In the present example, the operator presses these buttons on the touch screen display in this sequence: "Manifolds removed", "Ports blanked", "Fill door closed" and "Finish cycle". Upon receiving these inputs from the touch screen display, the controller 410 controls the vessel motor 140 to rotate the vessel 120 to the shredding position (S1113).

In some examples, a wash cycle is carried out once the vessel 120 has returned to the shredding position. The wash cycle may be as described above in relation to the waste cycle.

**Reject Cycle**

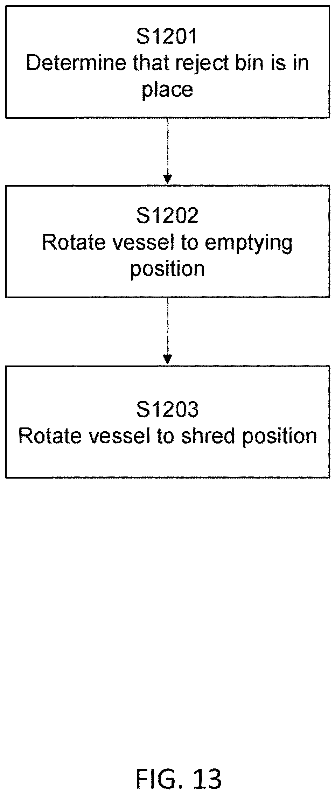

An example of a reject cycle process is shown in Figure 13. The option to start a reject cycle may be enabled when the controller 410 determines that there is an issue with shredding the waste within the vessel 120. For example, if the controller 410 determines that the torque of the blade arrangement motor 150 has exceeded a maximum torque value a predefined number of times, the controller 410 may enable the reject cycle.

In the present example, in response to receiving a selection of a "Reject Cycle" button from the touch screen display, the controller 410 will control the display to present a reject cycle screen. The operator puts the reject bin into position, and plugs in a reject bin identifier plug into an identifier socket. The control determines that the reject bin is in place (S1201) once it registers that the reject identifier plug is in position. This verification step ensures that waste will only be emptied from the vessel 120 when the reject bin is in place, reducing the risk of waste being released into the surrounding environment.

The controller 410 controls the touch screen display to turn the button "Reject bin in position" grey. In response to receiving a selection of the "Reject bin in position" button from the touch screen display, the controller 410 controls the touch screen display to turn the button "Reject" grey. In response to receiving a selection of the "Reject" button", the controller 410 controls the vessel motor 140 to rotate the vessel 120 to the emptying position (S1202). Waste is emptied from the vessel into the reject bin. The controller 410 may control the blade arrangement motor 150 to rotate for a predefined emptying time (e.g. 45 seconds) to help empty the vessel 120. Once the reject cycle has completed, the controller 410 will control the vessel motor 140 to rotate the vessel 120 to the shred position (S1203).

In some examples, a wash cycle is carried out once the vessel 120 has returned to the shredding position. The wash cycle may be as described above in relation to the waste cycle.

In various examples above, operation of the waste processing apparatus 1000 has been described as being controlled by a user. However, in alternative examples, the operation of the waste processing apparatus 1000 may be controlled by an external system (e.g. a testing or processing system which produces contaminated waste or biohazardous waste in use). Such automation further reduces that chances of users coming into contact with biohazardous waste, contaminants, or treatment fluids. In such cases, an automated loading device (e.g. a robotic arm, conveyor or other automated handling system or means) may remove waste from the testing system and load the waste into the waste processing vessel 120 of the waste processing apparatus 1000 without requiring human input. The waste processing apparatus 1000 may then process the waste according to a waste processing cycle and return the waste processing vessel 120 to the loading position so that it can accept another load of waste from the testing system.

Unless otherwise stated, each of the integers described may be used in combination with any other integer as would be understood by the person skilled in the art. Further, although all aspects of the disclosure preferably "comprise" the features described in relation to that aspect, it is specifically envisaged that they may "consist" or "consist essentially" of those features outlined in the claims. In addition, all terms, unless specifically defined herein, are intended to be given their commonly understood meaning in the art.

Further, in the discussion of the various examples, unless stated to the contrary, the disclosure of alternative values for the upper or lower limit of the permitted range of a parameter, is to be construed as an implied statement that each intermediate value of said parameter, lying between the smaller and greater of the alternatives, is itself also disclosed as a possible value for the parameter.

In addition, unless otherwise stated, all numerical values appearing in this application are to be understood as being modified by the term "about". Any means for providing a function in this disclosure may be provided in the form of an apparatus, device or system for performing that function, or configured to provide that function, including the examples specifically described.

Various modifications, whether by way of addition, deletion and/or substitution, may be made to all of the above-described embodiments to provide further embodiments, any and/or all of which are intended to be encompassed by the appended claims.

## Claims

1. A waste processing apparatus, comprising:

   a waste processing vessel for holding waste and treatment fluid during processing; and
   a fluid delivery system for delivering a treatment fluid to the waste processing vessel, the fluid delivery system comprising:

   a settling vessel located upstream of the waste processing vessel, the settling vessel defining an internal volume configured to retain a treatment fluid;
   a first fluid transfer means arranged and configured to transfer the treatment fluid from a fluid reservoir to the settling vessel; and
   a second fluid transfer means arranged and configured to deliver treatment fluid from the settling vessel to the waste processing vessel;
   wherein the fluid delivery system is configured such that treatment fluid is retained in the settling vessel for a period of time prior to delivery to the waste processing vessel.

2. The waste processing apparatus of claim 1, wherein the fluid delivery system further comprises a sensor arrangement configured to measure a parameter associated with the fluid in the settling vessel and to generate a signal based on the parameter.

3. The waste processing apparatus of claim 2, wherein the sensor arrangement comprises a plurality of level sensors.

4. The waste processing apparatus according to claim 3, wherein the settling vessel comprises at least two slots in a side wall thereof, each slot configured to receive a respective level sensor.

5. The waste processing apparatus of claim 4, wherein the at least two slots are arranged such that the respective level sensors are vertically aligned relative to each other with respect to a base region of the settling vessel.

6. The waste processing apparatus of any preceding claim, wherein the first fluid transfer means and/or the second fluid transfer means comprises a valve moveable to control flow of treatment fluid into and/or out of the settling vessel.

7. The waste processing apparatus of claim 2, wherein the fluid delivery system further comprises a controller configured to control flow of treatment fluid into and out of the settling vessel based on the signal from the sensor arrangement.

8. The waste processing apparatus of claim 7, wherein the controller is configured to determine whether a predetermined condition associated with the fluid in the settling vessel has been met based on the signal from the sensor arrangement and to generate a first output to instruct the second fluid transfer means to transfer the fluid from the settling vessel to the waste processing vessel based at least upon a determination that the predetermined condition has been met

9. The waste processing apparatus of claim 8, wherein the predetermined condition comprises a minimum settling time over which the treatment fluid has been present in the settling vessel.

10. The waste processing apparatus of claim 8 or claim 9, wherein the controller is configured to generate the first output based upon a further determination that a predetermined volume of fluid is present in the settling vessel.

11. The waste processing apparatus of any of claims 7 to 10, wherein the predetermined condition comprises a predetermined volume of treatment fluid present in the settling vessel.

12. The waste processing apparatus of claim 10 or claim 11, wherein the controller is configured to determine whether a volume of treatment fluid in the settling vessel is below the predetermined volume based on the signal from the sensor arrangement and in response thereto generate a second output to instruct the first fluid transfer means to deliver further treatment fluid into the settling vessel from the fluid reservoir.

13. A method of delivering a treatment fluid to a waste treatment system, the method comprising:

transferring a treatment fluid from a fluid reservoir to a settling vessel configured to retain the treatment fluid;
retaining the treatment fluid in the settling vessel for a period of time;
transferring the treatment fluid from the settling vessel to a waste treatment system after the period of time has elapsed.

14. A computer-readable medium comprising instructions which, when executed by a processor, cause the processor to: control a first fluid transfer means to transfer a treatment fluid from a fluid reservoir to a settling vessel configured to retain the fluid;

retain the treatment fluid in the settling vessel for a period of time; and
control a second fluid transfer means to transfer the treatment fluid from the settling vessel to a waste processing vessel after the period of time has elapsed.

15. A fluid delivery system for delivering a treatment fluid to a waste treatment system, the fluid delivery system comprising:

a settling vessel located upstream of the waste processing vessel, the settling vessel defining an internal volume configured to retain a treatment fluid;
a first fluid transfer means arranged and configured to transfer the treatment fluid from a fluid reservoir to the settling vessel;
a second fluid transfer means arranged and configured to deliver treatment fluid from the settling vessel to a waste treatment system;
wherein the fluid delivery system is configured to retain treatment fluid in the settling vessel for a period of time prior to delivering the treatment fluid to the waste processing vessel.

FIG. 1

FIG. 2

FIG. 3

FIG. 4A

FIG. 4B

FIG.5A

FIG. 5B

FIG. 6A

1534

1550

1550

1534a

FIG. 6B

| (a) | (b) | (c) | (d) |

FIG. 6C

FIG. 7A

FIG. 7B

FIG. 7C

FIG. 8

```
┌─────────────────────────────┐
│            S901             │
│     Control actuator to move │
│   shredding device into shred│
│           position          │
└─────────────────────────────┘
              │
              ▼
┌─────────────────────────────┐
│            S902             │
│  Control fluid delivery system to│
│    deliver treatment fluid into │
│        shredding device     │
└─────────────────────────────┘
              │
              ▼
┌─────────────────────────────┐
│            S903             │
│   Control shredding device to│
│   perform shredding function │
└─────────────────────────────┘
```

FIG. 9

```
┌─────────────────────────────────────┐
│              S1301                    │
│  Control first fluid transfer means to│
│   transfer treatment fluid to settling│
│               vessel                  │
└─────────────────────────────────────┘
                   │
                   ▼
┌─────────────────────────────────────┐
│              S1302                    │
│  Determine whether  predetermined    │
│   condition has been met based on    │
│          measured parameter          │
└─────────────────────────────────────┘
                   │
                   ▼
┌─────────────────────────────────────┐
│              S1303                    │
│  Control second fluid transfer means │
│  to transfer treatment fluid to waste│
│          processing vessel           │
└─────────────────────────────────────┘
```

# FIG. 10

S1001
Rotate vessel to fill position

S1002
Rotate vessel to shred position

S1003
Lower lid to cover vessel

S1004
Lock vessel in shred position

S1005
Deliver treatment fluid into vessel

S1006
Perform shredding function

FIG. 11A

```
┌─────────────────────────────┐
│         S1007               │
│ Terminate shredding function│
└─────────────────────────────┘
              │
              ▼
┌─────────────────────────────┐
│         S1008               │
│      Unlock vessel          │
└─────────────────────────────┘
              │
              ▼
┌─────────────────────────────┐
│         S1009               │
│       Raise lid             │
└─────────────────────────────┘
              │
              ▼
┌─────────────────────────────┐
│         S1010               │
│ Rotate vessel to emptying   │
│        position             │
└─────────────────────────────┘
              │
              ▼
┌─────────────────────────────┐
│         S1011               │
│ Control conveying means to  │
│   move shredded waste to    │
│        waste bin            │
└─────────────────────────────┘
              │
              ▼
┌─────────────────────────────┐
│         S1012               │
│ Rotate vessel to shredding  │
│        position             │
└─────────────────────────────┘
```

FIG. 11B

```
┌─────────────────────────────┐
│           S1101             │
│   Rotate vessel to emptying │
│          position           │
└─────────────────────────────┘
              │
              ▼
┌─────────────────────────────┐
│           S1102             │
│  Rotate vessel to fill      │
│          position           │
└─────────────────────────────┘
              │
              ▼
┌─────────────────────────────┐
│           S1103             │
│  Rotate vessel to shred     │
│          position           │
└─────────────────────────────┘
              │
              ▼
┌─────────────────────────────┐
│           S1104             │
│   Lower lid to cover vessel │
└─────────────────────────────┘
              │
              ▼
┌─────────────────────────────┐
│           S1105             │
│  Lock vessel in shred       │
│          position           │
└─────────────────────────────┘
              │
              ▼
┌─────────────────────────────┐
│           S1106             │
│  Deliver treatment fluid    │
│          into vessel        │
└─────────────────────────────┘
              │
              ▼
┌─────────────────────────────┐
│           S1107             │
│  Perform shredding function │
└─────────────────────────────┘
```

FIG. 12A

```
┌─────────────────────────────────┐
│            S1108                │
│   Terminate shredding function  │
└─────────────────────────────────┘
                 │
                 ▼
┌─────────────────────────────────┐
│            S1109                │
│         Unlock vessel           │
└─────────────────────────────────┘
                 │
                 ▼
┌─────────────────────────────────┐
│            S1110                │
│          Raise lid              │
└─────────────────────────────────┘
                 │
                 ▼
┌─────────────────────────────────┐
│            S1111                │
│    Rotate vessel to emptying    │
│           position              │
└─────────────────────────────────┘
                 │
                 ▼
┌─────────────────────────────────┐
│            S1112                │
│   Control conveying means to    │
│    move shredded waste to       │
│          waste bin              │
└─────────────────────────────────┘
                 │
                 ▼
┌─────────────────────────────────┐
│            S1113                │
│   Rotate vessel to shredding    │
│           position              │
└─────────────────────────────────┘
```

FIG. 12B

S1201
Determine that reject bin is in place

S1202
Rotate vessel to emptying position

S1203
Rotate vessel to shred position

FIG. 13

Europäisches
Patentamt
European
Patent Office
Office européen
des brevets

# EUROPEAN SEARCH REPORT

**Application Number**

EP 24 19 7117

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 116 574 A (PEARSON ERICH H [US]) 26 May 1992 (1992-05-26) * columns 6, 10; figure 1 * ----- | 1-15 | INV. B01D19/00 B01D21/30 B01D21/34 |
| X | GB 2 407 051 A (SAIT GRAHAM ANDREW [GB]) 20 April 2005 (2005-04-20) * pages 6,7; figure 2 * ----- | 1,13-15 | |

**TECHNICAL FIELDS SEARCHED (IPC)**

F17C
B01D

The present search report has been drawn up for all claims

| Place of search | Date of completion of the search | Examiner |
|---|---|---|
| Munich | 13 February 2025 | Tassinari, Francesca |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

..................................................................................................

& : member of the same patent family, corresponding
    document

EPO FORM 1503 03.82 (P4C01)

## ANNEX TO THE EUROPEAN SEARCH REPORT
## ON EUROPEAN PATENT APPLICATION NO.

EP 24 19 7117

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2025

| Patent document cited in search report | | Publication date | Patent family member(s) | | Publication date |
|---|---|---|---|---|---|
| US 5116574 | A | 26-05-1992 | US | 5116574 A | 26-05-1992 |
| | | | ZA | 916912 B | 29-04-1992 |
| GB 2407051 | A | 20-04-2005 | NONE | | |

EPO FORM P0459

For more details about this annex : see Official Journal of the European Patent Office, No. 12/82